(19) Europäisches Patentamt
European Patent Office
Office européen des brevets

(11) **EP 1 037 921 B1**

(12) # EUROPÄISCHE PATENTSCHRIFT

(45) Veröffentlichungstag und Bekanntmachung des Hinweises auf die Patenterteilung:
**24.09.2014 Patentblatt 2014/39**

(51) Int Cl.:
*C07K 14/505* [(2006.01)]     *C12N 15/16* [(2006.01)]
*C12N 15/85* [(2006.01)]     *C12N 5/10* [(2006.01)]
*A61K 38/18* [(2006.01)]

(21) Anmeldenummer: **98965756.4**

(22) Anmeldetag: **03.12.1998**

(86) Internationale Anmeldenummer:
**PCT/EP1998/007876**

(87) Internationale Veröffentlichungsnummer:
**WO 1999/028346 (10.06.1999 Gazette 1999/23)**

(54) **ERYTHROPOIETIN MIT HOHER SPEZIFISCHER AKTIVITÄT**

ERYTHROPOIETIN WITH HIGH SPECIFIC ACTIVITY

ERYTHROPOIETINE A ACTIVITE SPECIFIQUE MARQUEE

(84) Benannte Vertragsstaaten:
**AT BE CH DE DK ES FI FR GB GR IE IT LI LU NL PT SE**

(30) Priorität: **03.12.1997 DE 19753681**
**17.07.1998 EP 98113415**

(43) Veröffentlichungstag der Anmeldung:
**27.09.2000 Patentblatt 2000/39**

(73) Patentinhaber: **Roche Diagnostics GmbH**
**68305 Mannheim (DE)**

(72) Erfinder:
• **BURG, Josef**
**D-82362 Weilheim (DE)**
• **SELLINGER, Karl-Heinz**
**D-82362 Weilheim (DE)**
• **HASELBECK, Anton**
**D-82362 Weilheim (DE)**
• **KOLL, Hans**
**D-82362 Weilheim (DE)**

(74) Vertreter: **Waschbüsch, Klaus et al**
**F. Hoffmann-La Roche AG**
**Patent Department (CLP)**
**Grenzacherstrasse 124**
**4070 Basel (CH)**

(56) Entgegenhaltungen:
EP-A1- 0 267 678     EP-A1- 0 754 703
EP-A2- 0 475 354     WO-A1-95/12684
WO-A1-96/35718     US-A- 5 047 335

• TAKEUCHI M ET AL: "RELATIONSHIP BETWEEN SUGAR CHAIN STRUCTURE AND BIOLOGICAL ACTIVITY OF RECOMBINANT HUMAN ERYTHROPOIETIN PRODUCED IN CHINESE HAMSTER OVARY CELLS" PROCEEDINGS OF THE NATIONAL ACADEMY OF SCIENCES OF USA, Bd. 86, Oktober 1989, Seiten 7819-7822, XP002053701 in der Anmeldung erwähnt
• FUKUDA M N ET AL: "SURVIVAL OF RECOMBINANT ERYTHROPOIETIN IN THE CiRCULATION: THE ROLE OF CARBOHYDRATES" BLOOD, Bd. 73, Nr. 1, Januar 1989, Seiten 84-89, XP000199651 in der Anmeldung erwähnt
• WATSON E ET AL: "STRUCTURE DETERMINATION OF THE INTACT MAJOR SIALYLATED OLIGOSACCHARIDE CHAINS OF RECOMBINANT HUMAN ERYTHROPOIETIN EXPRESSED IN CHINESE HAMSTER OVARY CELLS" GLYCOBIOLOGY, Bd. 4, Nr. 2, 1. Januar 1994, Seiten 227-237, XP000576965
• NIMTZ M ET AL: "STRUCTURES OF SIALYLATED OLIGOSACCHARIDES OF HUMAN ERYTHROPOIETIN EXPRESSED IN RECOMBINANT BHK-21 CELLS" EUROPEAN JOURNAL OF BIOCHEMISTRY, Bd. 213, Nr. 1, 1. April 1993, Seiten 39-56, XP000576796

• HOKKE C H ET AL: "STRUCTURAL ANALYSIS OF THE SIALYLATED N- AND O-LINKED CARBOHYDRATE CHAINS OF RECOMBINANT HUMAN ERYTHROPOIETIN EXPRESSED IN CHINESE HAMSTER OVARY CELLS. SIALYLATION PATTERNS AND BRANCH LOCATION OF DIMERIC N-ACETYLLACTOSAMINE UNITS" EUROPEAN JOURNAL OF BIOCHEMISTRY, Bd. 228, März 1995, Seiten 981-1008, XP000199646

• CHOI D ET AL: "Erythropoietin: physico- and biochemical analysis" JOURNAL OF CHROMATOGRAPHY B: BIOMEDICAL APPLICATIONS, Bd. 687, Nr. 1, 6. Dezember 1996, Seite 189-199 XP004062816

**Beschreibung**

Die Erfindung betrifft neue EPO-Zusammensetzungen mit hoher spezifischer Aktivität, die durch einen hohen Gehalt an N-Acetyl-Lactosamin-Einheiten oder/und tetraantennären Verzweigungen in der Kohlenhydratstruktur gekennzeichnet sind. Weiterhin betrifft die Erfindung ein pharmazeutisches Präparat, welches eine erfindungsgemäße EPO-Zusammensetzung enthält.

Erythropoietin (EPO) ist ein humanes Glycoprotein, welches die Produktion von roten Blutzellen stimuliert. EPO kommt im Blutplasma von gesunden Personen nur in sehr geringen Konzentrationen vor, so daß eine Bereitstellung in größeren Mengen auf diese Weise nicht möglich ist.

EP-B1-0 148 605 und EP-B1-0 205 564 beschreiben die Herstellung von rekombinantem humanen EPO in CHO-Zellen. Das in EP-B1-0 148 605 beschriebene EPO weist ein höheres Molekulargewicht als urinäres EPO und keine O-Glykosilierung auf. Das in EP-B1-0 205 564 beschriebene EPO aus CHO-Zellen ist mittlerweile in großen Mengen und in reiner Form verfügbar.

Weiterhin ist die Gewinnung von humanem EPO aus dem Urin von Patienten mit aplastischer Anämie bekannt (Miyake et al., J. Biol. Chem. 252 (1977), 5558-5564).

Rekombinantes und urinäres EPO wird als Gemisch verschiedener Isoformen gewonnen, von denen bekannt ist, daß sie sich in ihrem Sialylierungsgrad unterscheiden. Diese EPO-Isoformen weisen unterschiedliche isoelektrische Punkte auf und können durch isoelektrische Fokussierung bzw. Kapillarelektrophorese aufgetrennt werden (siehe Tsao et al., Biotech. Bioeng. 40 (1992), 1190-1196; Nieto et al., Anal. Commun. 33 (1996), 425-427; Tran et al., J. Chromatogr. 542 (1991), 459-471; Bietot et al., J. Chromatogr. 759 (1997), 177-184; Watson et al. Anal. Biochem. 210 (1993), 389-393). Die Isoformen mit der höchsten Anzahl von Sialinsäuren weisen die höchste spezifische Aktivität auf, während die mit der niedrigsten Anzahl die geringste Aktivität besitzen (siehe z.B. Imai et al., Eur. J. Biochem. 194 (1990), 457-462; EP-A-0 428 267).

Takeuchi et al., (Proc. Natl. Acad. Sci. USA 86 (1989), 7819-7822) beschreiben einen Zusammenhang der biologischen Aktivität mit dem Sialinsäuregehalt und dem Verhältnis von bi- und tetraantennären Kohlenhydratstrukturen. Takeuchi et al. kommen weiterhin zur Schlußfolgerung, daß die in den EPO-Kohlenhydratstrukturen vorhandenen N-Acetyl-Lactosamin-Einheiten nicht mit der biologischen Aktivität korrelieren.

Fukuda et al. (Blood 73 (1989), 84-89) befassen sich mit der Geschwindigkeit der Elimination von EPO aus dem Blutkreislauf, die wesentlich zur biologischen Aktivität beiträgt, und kommen zu dem Schluß, daß EPO mit einer größeren Anzahl an N-Acetyl-Lactosamin-Einheiten schneller aus dem Kreislauf entfernt wird als EPO ohne Lactosamin-Einheiten. Morimoto et al. (Glycoconjugate J. 13 (1996), 1093-1120) beschreiben die Auftrennung von EPO-Isoformen mittels Mono-Q-Chromatographie, so daß die einzelnen Fraktionen jeweils aus nur noch wenigen Isoformen bestehen. Die mit diesen Fraktionen durchgeführten Untersuchungen zeigen eine Gleichverteilung aller Strukturen in allen Fraktionen. Es wird keine Korrelation des Gehalts an bi- oder triantennären Strukturen oder des Gehalts an N-Acetyl-Lactosamin-Einheiten mit der spezifischen Aktivität gefunden.

Somit geht aus dem genannten Stand der Technik hervor, daß eine generelle Korrelation der biologischen Aktivität mit der Zuckerstruktur besteht, insbesondere im Hinblick auf den Gehalt an Sialinsäuren. Es findet sich jedoch keinerlei Hinweis, daß der Anteil an tetraantennären Strukturen oder/und der Anteil an N-Acetyl-Lactosamin eine direkte Korrelation mit der biologischen Aktivität besitzt.

Überraschenderweise wurde bei der Aufreinigung von EPO-Präparationen festgestellt, daß eine Erhöhung des Anteils an tetraantennären Kohlenhydratstrukturen oder/und N-Acetyl-Lactosamin-Einheiten in der Kohlenhydratstruktur zu einer signifikanten Verbesserung der spezifischen biologischen Aktivität führt. Dies trifft insbesondere zu bei der Herstellung von EPO in einer humanen Zellinie gemäß der europäischen Anmeldung 97 112 640.4.

Vergleichende Aktivitätsuntersuchungen einzelner EPO-Präparationen oder EPO-Isoformen, deren Kohlenhydratstruktur sich im wesentlichen nur im Gehalt der N-Acetyl-Lactosamin-Einheiten (LE-Einheiten) unterscheidet, zeigen eine signifikant höhere Aktivität für die Präparationen oder Isoformen mit dem höheren Anteil an N-Acetyl-Lactosamin-Einheiten bei gleichen Sialinsäuregehalt und bei etwa gleicher Antennärität. Unter Antennärität wird in diesem Zusammenhang der relative, mittlere Gehalt (in %) an bi-, tri- bzw. tetraantennären N-gebundenen Kohlenhydratketten der EPO-Präparationen bzw. der isolierten EPO-Isoformen bezüglich der Gesamtzahl N-gebundener Kohlenhydratketten verstanden. Außerdem wurde gefunden, daß gerade in Präparationen oder Isoformen mit einem erhöhten Anteil an triantennären Strukturen der Gesamtgehalt an Lactosamin-Einheiten, für die in vivo Aktivität von erheblicher Bedeutung ist. Durch eine Erhöhung des Gesamtgehalts an N-Acetyl-Lactosamin-Einheiten, z. B. in Form von zusätzlichen Verlängerungen der Core-Struktur mit LE-Einheiten (sog. Repeats), kann die biologische Aktivität erheblich gesteigert werden. Weiterhin wurde festgestellt, daß durch Erhöhung des Anteils an tetraantennären Strukturen eine Verbesserung der biologischen Aktivität erreicht werden kann.

Ist man folglich bestrebt, ein EPO-Präparat mit möglichst hoher spezifischer Aktivität und in hoher Ausbeute zu erzeugen, so ist bei der Auswahl der Aufreinigungsschritte, der Produktionszellen oder/und bei deren Kultivierung auf einen möglichst hohen Anteil von tetraantennären Kohlenhydratstrukturen oder/und einen möglichst hohen Gehalt an N-Acetyl-Lactosamin-Einheiten zu optimieren.

Ein erster Aspekt der Erfindung betrifft eine EPO-Zusammensetzung, die im wesentlichen aus glykosilierten EPO-Molekülen besteht, die eine Anzahl von durchschnittlich mindestens 3,7, vorzugsweise von mindestens 4,O besonders bevorzugt von mindestens 4,3 und am meisten bevorzugt von mindestens 4,5 N-Acetyl-Lactosamin-Einheiten bezogen auf die mittlere Zusammensetzung pro N-gebundener Kohlenhydratkette des EPO-Moleküls bzw. eine Anzahl von mindestens 11,1, vorzugsweise von mindestens 12,0, besonders bevorzugt von mindestens 13,0 und am meisten bevorzugt von mindestens 13,5 N-Acetyl-Lactosamin-Einheiten bezogen auf alle 3 N-gebundenen Kohlenhydratstrukturen (Gesamt-N-Glycosilierung) des EPO-Moleküls enthalten.

Die EPO-Moleküle sind das Produkt einer Expression endogener DNA in humanen Zellen. Der Anteil von Kohlenhydratketten mit N-Acetyl-Lactosamin-Repeats bezogen auf die Gesamtzahl von Kohlenhydratketten ist mindestens 10%. Die EPO-Zusammensetzung weist eine spezifische Aktivität in vivo von mindestens 175 000 IU/mg Protein auf.

Die EPO-Zusammensetzung besteht vorzugsweise im wesentlichen aus glykosilierten EPO-Molekülen, die einen Wert für das Produkt aus der durchschnittlichen Gesamtzahl von N-Acetyl-Lactosamin-Einheiten pro EPO Molekül multipliziert mit dem mittleren Sialinsäuregehalt pro Molekül EPO von mindestens 130, vorzugsweise von mindestens 135, besonders bevorzugt von mindestens 140 und am meisten bevorzugt mindestens 160 aufweisen.

Die Angabe "im wesentlichen" bedeutet in diesem Zusammenhang, daß die gewünschten EPO-Moleküle in einem Anteil von vorzugsweise mindestens 80%, besonders bevorzugt von mindestens 90% und am meisten bevorzugt von mindestens 95% bezogen auf die Gesamtzahl der EPO-Moleküle in der Zusammensetzung vorhanden sind.

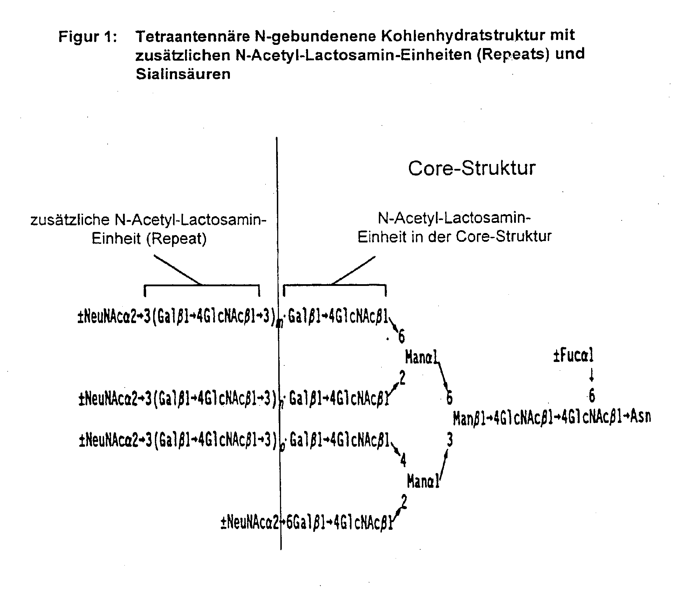

Die Höchstgrenze des Anteils an tetraantennären Strukturen kann bis zu 100% der gesamten Kohlenhydratketten reichen, wobei jede tetraantennäre Struktur 4 N-Acetyl-Lactosamin-Einheiten in der core-Struktur des N-gebundenen Zuckers enthält. Zusätzliche N-Acetyl-Lactosamin-Einheiten als sogenannte Repeats, die als Verlängerungen an der core-Struktur vorkommen, können die Zahl der N-Acetyl-Lactosamin-Einheiten sowohl pro Kohlenhydratstruktur, als auch in der Gesamtglycosilierung erhöhen. Die Anzahl an N-Acetyl-Lactosamin-Einheiten pro Glykosilierungsstelle (d.h. pro N-gebundener Kohlenhydratstruktur) kann somit bis zu 6 (tetraantennäre Struktur und 2 zusätzliche N-Acetyl-Lactösamin-Einheiten in Form von Repeats) betragen (vgl. Fig. 1) oder - bei Strukturen mit mehr als 2 zusätzlichen N-Acetyl-Lactosamin-Einheiten - sogar noch höher sein. Bezogen auf die Gesamtglykosilierung (3 N-gebundene Kohlenhydratstrukturen) kann die Anzahl der N-Acetyl-Lactosamin-Einheiten bis zu 18 oder höher betragen.

Die erfindungsgemäße Zusammensetzung kann aus einer oder mehreren Isoformen, d.h. EPO-Molekülen mit unterschiedlichen isoelektrischen Punkten bei der isoelektrischen Fokussierung, bestehen. Vorzugsweise umfasst die erfindungsgemäße Zusammensetzung ein Gemisch aus mindestens 2, z.B. aus 2 bis 5 Isoformen, insbesondere ein Gemisch aus 3 oder 4 Isoformen.

Die. spezifische Aktivität der erfindungsgemäßen Zusammensetzung ist mindestens 175 000 IU/mg, insbesondere mindestens 200 000 IU/mg in vivo (normozythämische Maus). Besonders bevorzugt ist die spezifische Aktivität im Bereich von etwa 200 000 bis 400 000 IU/mg oder 450 000 IU/mg Protein, am meisten bevorzugt im Bereich von 250 000 bis 400 000 IU/mg oder 450 000 IU/mg Protein.

Der mittlere Sialinsäuregehalt bzw. die mittlere Anzahl der Sialinsäurereste pro Molekül EPO beträgt in der erfindungsgemäßen Zusammensetzung vorzugsweise 11 bis 14, besonders bevorzugt mindestens 11,5 und am meisten bevorzugt mindestens 12,5.

Die erfindungsgemäße Zusammensetzung besteht aus EPO-Molekülen, welche das Produkt einer Expression endogener DNA nach Genaktivierung in humanen Zellen, z.B. in immortalisierten Zellinien wie etwa Namalwa (Nadkarni et al., Cancer 23 (1969), 64-79), HT1080 (Rasheed et al., Cancer 33 (1973), 1027-1033) oder HeLa S3 (Puck et al., J. Exp. Meth. 103 (1956), 273-284) sind. Derartige Verfahren sind in der europäischen Patentanmeldung 97 112 640.4 beschrieben.

Weitere wichtige Parameter für die biologische Aktivität von EPO sind der Anteil von Kohlenhydratketten mit Repeats, d.h. zusätzlichen N-Acetyl-Lactosamin-Einheiten, bezogen auf die Gesamtzahl von N-gebundenen Kohlenhydratketten sowie der Wert des Produkts dieses Repeat-Anteils mit dem Anteil von tetraantennären Kohlenhydratketten bezogen auf die Gesamtzahl von Kohlenhydratketten. Bei EPO aus CHO-Zellen liegt der Repeat-Anteil vorzugsweise bei mindestens 30%, besonders bevorzugt bei mindestens 35% und am meisten bevorzugt bei mindestens 40%. Bei EPO aus humanen Zellen wie etwa HeLa-Zellen liegt der Repeat-Anteil bei vorzugsweise mindestens 10%, besonders bevorzugt bei mindestens 12% und am meisten bevorzugt bei mindestens 14%. Dementsprechend liegt bei EPO aus CHO-Zellen der Wert für das Produkt aus dem Anteil von Kohlenhydratketten mit N-Acetyl-Lactosamin-Repeats bezogen auf die Gesamtzahl von Kohlenhydratketten und dem Anteil von tetraantennären Strukturen bezogen auf die Gesamtzahl von Kohlenhydratketten vorzugsweise bei mindestens 2400, besonders bevorzugt bei mindestens 2800 und am meisten bevorzugt bei mindestens 3400. Bei EPO aus humanen Zellen liegt der Wert vorzugsweise bei mindestens 800, besonders bevorzugt bei mindestens 960 und am meisten bevorzugt bei mindestens 1 100.

Besonders bevorzugt wird eine EPO-Zusammensetzung verwendet, die durch Kultivierung von EPO-Produktionszellen in einem Kulturmedium mit geringem Serumgehalt, z.B. maximal 1 % (v/v) oder insbesondere in einem serumfreien Kulturmedium hergestellt wurde (vgl. hierzu WO 96/35718). Beispiele für geeignete Kulturmedien sind RPMI 1640 oder DMEM.

In einem weiteren Aspekt wird die erfindungsgemäße EPO-Zusammensetzung als pharmazeutisches Präparat gegebenenfalls zusammen mit üblichen pharmazeutischen Verdünnungs-, Hilfs- und Trägermitteln formuliert. Die erfindungsgemäße EPO-Zusammensetzung, die zur Herstellung eines pharmazeutischen Präparats eingesetzt werden kann, hat eine Reinheit von vorzugsweise mindestens 99% und besonders bevorzugt von mindestens 99,9% bei Bestimmung durch Reverse Phase-HPLC (z.B. auf einer Vydac C4-Säule) oder/und Größenausschlußchromatographie (z.B. auf einen TSK 2000SW Ultrapac-Säule).

Außerdem weist die erfindungsgemäße Zusammensetzung einen DNA-Gehalt von vorzugsweise < 10 pg, besonders bevorzugt < 5 pg und am meisten bevorzugt < 1 pg DNA pro 10 000 IU Protein auf. Außerdem ist die erfindungsgemäße Zusammensetzung vorzugsweise weitgehend frei von bakteriellen Verunreinigungen ( < 1 CFU/ml) und Endotoxinen ( < 1 EU/10 000 IU Protein).

Die Bestimmung des DNA-Gehalts kann durch einen Hybridisierungstest mit radioaktiver oder fluoreszenzmarkierter DNA erfolgen. Als Sonden-DNA wird z.B. kommerziell erhältliche aufgereinigte Human-DNA verwendet. Die Human-DNA kann zudem als Standard für den Test eingesetzt werden. Die untere Nachweisgrenze eines solchen Hybridisierungstests beträgt etwa 0,3 pg/10 000 IU EPO. Der Keim- und Endotoxingehalt in der EPO-Präparation kann nach standardisierten Methoden bestimmt werden, wie sie in Pharm. Eu. oder USP beschrieben sind.

Eine EPO-Zusammensetzung, die vorzugsweise die erfindungsgemäß erwünschten Merkmale aufweist, ist erhältlich durch mindestens eine der folgenden Maßnahmen:

(a) Auswahl einer geeigneten humanen Produktionszellinie; welche in der Lage ist, Kohlenhydratketten mit einem hohen Anteil tetraantennärer Strukturen oder/und N-Acetyl-Lactosamin-Einheiten zu erzeugen,

(b) Auswahl von geeigneten Kultivierungsbedingungen bei der Zellkultur, um Kohlenhydratketten mit einem hohen Anteil tetraantennärer Struktur oder/und N-Acetyl-Lactosamin-Einheiten zu erzeugen und

(c) Abtrennung unerwünschter Bestandteile aus einer bekannten Zusammensetzung von EPO-Molekülen aus humanen Zellen unter Anreicherung von EPO-Molekülen, die Kohlenhydratketten mit einem hohen Anteil tetraantennärer Strukturen oder/und N-Acetyl-Lactosamin-Einheiten enthalten.

Maßnahme (a) umfaßt die Auswahl einer geeigneten Produktionszelle. Einerseits kann man hier humane Zellen verwenden, von denen bekannt ist, daß sie eine Tendenz aufweisen, die gewünschten Kohlenhydratkettenstrukturen mit großer Ausbeute herzustellen. Beispiele für solche Zellinien sind humane Zellinien wie HeLa, Namalwa, HT1080 oder davon abgeleitete Zellinien. Besonders bevorzugt sind HeLaS3-Zellen.

Andererseits können geeignete Produktionszellen auch gezielt erzeugt werden, indem bestimmte Glykosilierungsenzyme in der Zelle überexprimiert werden, z.B. durch rekombinante Expression oder/und durch endogene Genaktivierung. Beispiele solcher Glykosilierungsenzyme sind Sialyltransferasen, N-Acetyl-Glucosaminyltransferasen und Galactosyltransferasen.

Maßnahme (b) umfaßt die Auswahl geeigneter Kultivierungsbedingungen bei der Zellkultur. In einer ersten Ausführungsform der Erfindung umfaßt Maßnahme (b) die Zugabe einer Mischung von mindestens zwei und vorzugsweise mindestens drei Kohlenhydraten zum Kulturmedium. Die Kohlenhydrate werden vorzugsweise aus Mono- und Disacchariden wie etwa Glucose, Glucosamin, Ribose, Fructose, Galactose, Mannose, Saccharose, Lactose, Mannose-1-Phosphat, Mannose-1-Sulfat und Mannose-6-Sulfat ausgewählt. Beispielsweise sind Nährmedien geeignet, die Glucose oder/und Mannose oder/und Galactose enthalten. Besonders gute Ergebnisse wurden mit Nährmedien erzielt, die eine Mischung von Glucose, Galactose und Mannose, beispielsweise im Masseverhältnis von 1:(0,5-3):(1-5) und insbesondere von 1:(0,7-2,4):(1,8-4,0) enthalten, wobei die Kohlenhydrate jeweils besonders bevorzugt in der D(+)-Form eingesetzt werden. Die Gesamtkonzentration aller Zucker liegt während der Fermentation vorzugsweise in einem Bereich von 0,1 bis 10 g/l, besonders bevorzugt in einem Bereich von 2 bis 6 g/l im Kulturmedium. Vorzugsweise erfolgt die Zugabe der Kohlenhydratmischung abhängig vom jeweiligen Bedarf der Zellen wie im folgenden weiter ausgeführt wird.

Gemäß einer weiteren bevorzugten Ausführungsform umfaßt Maßnahme (b) die kontrollierte und vorzugsweise bedarfsgerechte Zugabe von Nährstoffen, umfassend mindestens eine für die kultivierte Zellinie essentielle Aminosäure oder/und mindestens ein Kohlenhydrat, abhängig vom jeweiligen Bedarf der Zellen. Auf diese Weise wird selbst bei einer Hochzelldichtefermentation (Zelldichte bei der Ernte > 10 x $10^5$ Zellen/ml und vorzugsweise > 20 x $10^5$ Zellen/ml) in Großfermentern (Volumen > 1 l, z.B. 50 - 10.000 l) eine deutlich verbesserte Glykosilierung erhalten. Hierzu wird kontinuierlich oder in geeigneten Zeitintervallen, z.B. mindestens einmal täglich, die Konzentration von Parametern bestimmt, die in Korrelation zum Nährstoffbedarf der Zellen stehen und deren Verbrauchsraten ermittelt. Auf diese Weise können die für den Bedarf der Zellen benötigten Nährstoffe quantitativ oder/und qualitativ ermittelt werden. Solche Parameter können Nährstoffe oder Stoffwechselprodukte der Zellen sein, wie etwa die Glutamin-, die Ammonium-, die Glucose- oder/und die Lactatkonzentration, insbesondere die Glutamin-Konzentration.

Die gemäß diesem Aspekt der Erfindung zugegebenen Nährstoffe umfassen essentielle Aminosäuren, z.B. Glutamin oder/und Tryptophan, oder/und Kohlenhydrate, sowie vorzugsweise weiterhin nichtessentielle Aminosäuren, Vitamine, Spurenelemente, Salze oder/und Wachstumsfaktoren, z.B. Insulin. Besonders bevorzugt umfassen die Nährstoffe mindestens eine essentielle Aminosäure und mindestens ein Kohlenhydrat. Diese Nährstoffe werden vorzugsweise in gelöstem Zustand dem Kulturmedium zudosiert. Die Nährstofflösungen enthalten vorzugsweise zumindest Glutamin und Kohlenhydrate, insbesondere eine Mischung aus mindestens 2 Kohlenhydraten wie zuvor genannt. Besonders bevorzugt wird eine Mischung von Glucose, Galactose und Mannose eingesetzt. Weiterhin ist bevorzugt, daß die Zugabe der Nährstoffe über die gesamte Wachstumsphase der Zellen bedarfsgerecht, d.h. abhängig von der im Kulturmedium gemessenen Konzentration der ausgewählten Parameter erfolgt.

Das Mengenverhältnis von Glutamin zu Kohlenhydraten in der Nährstofflösung wird vorzugsweise so gewählt, daß es dem Verbrauchsverhältnis im Fermenter im wesentlichen entspricht. Auf diese Weise kann eine weitgehend gleichbleibende Konzentration einzelner Substrate im Fermenter erreicht werden. Vorzugsweise wird die Konzentration von Glutamin auf einem Wert gehalten, der < 150 mg/l im Kulturmedium ist und die Entstehung einer Ammoniumkonzentration $\geq 2,3$ mmol/l im Kulturmedium verhindert. Die Gesamtkonzentration der Zucker liegt während der Fermentation - wie bereits ausgeführt - vorzugsweise in einem Bereich von 0,1 bis 10 g/l, besonders bevorzugt in einem Bereich von 2 bis 6 g/l Kulturmedium.

Die verwendete Nährstofflösung enthält ein Masseverhältnis von Glutamin zu Zuckern, das vorzugsweise im Bereich von 1:3 bis 20 und besonders bevorzugt von 1:5 bis 15, bezogen auf die gesamten Zucker, liegt. Bei Verwendung einer Nährstofflösung, die Glutamin sowie die drei Zucker Glucose, Galactose und Mannose enthält, liegt das Masseverhältnis von Glutamin zu den Zuckern vorzugsweise bei 1:(1 bis 3):(1 bis 5):(2 bis 8) und besonders bevorzugt bei 1:(1,5 bis 2,2):(1,5 bis 3,6):(4 bis 6).

Die Kultivierung wird vorzugsweise als wiederholt satzweiser Betrieb durchgeführt, wobei nach einer Wachstumsphase ein Teil der Kulturbrühe geerntet wird und der Rest der Kulturbrühe im Fermenter bleibt, der anschließend wieder bis zum Arbeitsvolumen mit frischem Medium befüllt wird. Durch das erfindungsgemäße Verfahren kann glykosiliertes EPO in sehr hohen Ausbeuten geerntet werden. So beträgt die Konzentration zum Erntezeitpunkt beispielsweise mindestens 30 mg und insbesondere mindestens 40 mg EPO pro l Kulturmedium.

Ebenso wird ein Verfahren zur Gewinnung von EPO aus eukaryontischen Zellen offenbart, wobei die eukaryontischen Zellen in einem geeigneten Medium kultiviert und das EPO aus dem Kulturüberstand gewonnen wird, wobei das Verfahren dadurch gekennzeichnet ist, daß die Kultivierung bei einer Temperatur von $\leq 36\ °$ C, vorzugsweise zwischen 30 und 35,5°C und besonders bevorzugt zwischen 33 und 35,0°C erfolgt. Überraschenderweise wurde nämlich festgestellt, daß durch Temperatursenkung bei der Kultivierung der Anteil an EPO mit gewünschter Glykosilierung deutlich erhöht wird.

Maßnahme (c) umfaßt die Abtrennung unerwünschter Bestandteile aus einer bekannten EPO-Zusammensetzung, die in ihrer Kohlenhydratstruktur die Spezifikationen der vorliegenden Anmeldung nicht erfüllt. Dies kann beispielsweise erfolgen durch chromatographische Aufreinigung der EPO-Präparationen, z.B. durch Affinitätschromatographie an Triazinfarbstoff-Gelen, vorzugsweise Cibacron Blue-Farbstoffgelen. Eine weitere Abtrennung unerwünschter Bestandteile kann auch mit hydrophober Interaktions-chromatographie und Reversed Phase Chromatographie durchgeführt werden. Dafür geeignete Liganden sind Butyl-, Pentyl-, Octyl- , Octadecyl- und Phenyl-Reste. Der Reversed Phase-Chromatographie-Schritt wird vorzugsweise bei einem pH-Wert im Bereich von 6,0 bis 8,5 und besonders bevorzugt von 7,0 bis 8,0 durchgeführt. Geeignete Eluenten sind beispielsweise Acetonitril, Ethanol oder Isopropanol, vorzugsweise Acetonitril.

Mit Kapillarzonenelektrophorese-Analytik (CZE) können geeignete Fraktionen ermittelt, gepoolt und schließlich weiterverarbeitet werden. Außerdem kann unter Verwendung von Lectinen aus Tomaten oder Kartoffeln (Merkle, Cummings, J. Biol. Chem. 262 (1987), 8179-8189) eine direkte Anreicherung von EPO-Molekülen mit einem hohen Anteil an N-Acetyl-Lactosamin-Einheiten erfolgen. Bevorzugt werden solche Lectine z.B. in immobilisierter Form eingesetzt.

Weiterhin wird die vorliegende Erfindung durch die nachfolgenden Figuren und Beispiele erläutert.

Es zeigen:

Figur 1:     die Darstellung einer tetraantennären Kohlenhydratstruktur mit zusätzlichen N-Acetyl-Lactosamin-Einheiten (Repeats) und Sialinsäuren,

Figur 2:     den relativen Anteil einzelner EPO-Isoformen in Abhängigkeit von den dem Kulturmedium zugesetzten Kohlenhydraten,

Figur 3:     die biologische Aktivität von EPO-Präparaten in Abhängigkeit von den dem Kulturmedium zugesetzten Kohlenhydraten und

Figur 4:     die biologische Aktivität von EPO-Isoformen in Abhängigkeit vom Anteil der N-Acetyl-Lactosamin-Repeat-Einheiten.

**Beispiel 1 Aufreinigung von EPO aus Kulturüberständen von Zellinien**

Im wesentlichen wurden zur Aufreinigung von EPO aus Zellkulturüberständen von humanen Zellen oder CHO-Zellen zwei Methoden verwendet, die sich in Anzahl und Prinzip der Chromatographieschritte unterschieden und abhängig von der Zusammensetzung des Mediums und der EPO-Konzentration eingesetzt wurden:

| | |
|---|---|
| Methode 1: | 1. Schritt: Blue-Sepharose-Säule |
| | 2. Schritt: Butyl-Sepharose-Säule |
| | 3. Schritt: Hydroxyapatit-Säule |
| | 4. Schritt: Aufkonzentrierung |
| Methode 2: | 1. Schritt: Blue-Sepharose-Säule |
| | 2. Schritt: Hydroxyapatit-Säule |
| | 3. Schritt: Aufkonzentrierung |
| | (alternativer 3. Schritt: RP-HPLC) |

Beispiel für eine Aufreinigung eines Hela S3-Zellkulturüberstandes mit 2 % (v/v) fötalem Kälberserum (FKS) nach Methode 1:

### 1. Blue Sepharose-Säule:

Eine 5 ml Hi-Trap-Blue-Säule (Blue-Sepharose-Fertigsäule von Pharmacia) wurde mit mindestens 5 Säulenvolumina (SV) Puffer A (20 mM Tris-HCl, pH, 7,0; 5 mM $CaCl_2$; 100 mM NaCl) äquilibriert. Anschließend wurden 70 ml Hela-Zellüberstand (ca. 245 $\mu$g EPO und 70-100 mg Gesamtprotein enthaltend) über Nacht bei einem Fluß von 0,5 ml/min im Kreislaufverfahren aufgezogen.

Die Säule wurde mit mindestens 5 SV Puffer B (20mM Tris-HCl, pH 7,0; 5 mM $CaCl_2$; 250 mM NaCl) und mindestens 5 SV Puffer C (20mM Tris-HCl, pH 7,0; 0,2 mM $CaCl_2$, 250 mM NaCl) bei 0,5 ml/min gewaschen. Der Wascherfolg wurde über die Messung des Proteingehalts bei OD280 verfolgt.

Die Elution von EPO erfolgte mit Puffer D (100 mM Tris-HCl, pH 7,0; 0,2 mM $CaCl_2$; 2 M NaCl) bei einem Fluß von 0,5 ml/min. Die Elutionslösung wurde in 1-2 ml Fraktionen gesammelt.

Der EPO-Gehalt der Fraktionen, der Waschlösungen und des Durchlaufs wurden über Reverse-Phase (RP)-HPLC durch Auftrag eines Aliquots auf eine POROS R2/H-Säule (Boehringer Mannheim) bestimmt. Alternativ wurde zur qualitativen Identifizierung EPO-haltiger Fraktionen ein immunologischer Dotblot durchgeführt.

EPO-haltige Fraktionen der Elution (8-12 ml) wurden gepoolt und auf eine Butyl-Sepharose-Säule aufgetragen.

Die Ausbeute nach der Blue-Sepharose-Säule betrug ca. 175 $\mu$g EPO (entspricht ca. 70%). Im Allgemeinen betrug die Ausbeute nach Blue-Sepharose zwischen 50-75%.

### 2. Butyl-Sepharose-Säule (Hydrophobe-Interaktions-Chromatographie)

Eine selbsthergestellte 2-3 ml Butyl-Sepharose-Säule (Material: Toyopearl Butyl S650) wurde mit mindestens 5 SV Puffer D (100 mM Tris-HCl, pH 7,0; 0,2 mM $CaCl_2$; 2 M NaCl) äquilibriert und anschließend der EPO-haltige Blue-Sepharose-Pool aus 1 . (ca. 150 $\mu$g EPO) bei einem Fluß von 0,5 ml/min aufgezogen.

Die Säule wurde mit mindestens 5 SV Puffer E (20 mM Tris-HCl, pH 7,0; 2 M NaCl und 10 % Isopropanol) bei 0,5 ml/min gewaschen. Der Wascherfolg wurde über die Messung des Proteingehalts bei OD280 verfolgt.

Die Elution von EPO erfolgte mit Puffer F (20 mM Tris-HCl, pH 7,0; 2 M NaCl und 20 % Isopropanol) bei Raumtemperatur und einem Fluß von 0,5 ml/min. Die Elutionslösung wurde in 1-2 ml Fraktionen gesammelt.

Der EPO-Gehalt der Fraktionen, der Waschlösungen und des Durchlaufs wurden über RP-HPLC durch Auftrag eines Aliquots auf eine POROS R2/H-Säule bestimmt. Alternativ wurde zur qualitativen Identifizierung EPOhaltiger Fraktionen ein immunologischer Dotblot durchgeführt. EPO-haltige Fraktionen der Elution (10-15 ml) wurden gepoolt und auf eine Hydroxy-apatit-Säule aufgetragen.

Die Ausbeute der Butyl-Sepharose-Säule betrug ca. 130 $\mu$g EPO (entspricht ca. 85 %). Im Allgemeinen betrug die Ausbeute der Butyl-Sepharose zwischen 60-85% vom Auftrag der Blue-Sepharose-Pools.

### 3. Hydroxyapatit-Säule

Eine 5 ml Hydroxyapatit-Säule (Econo-Pac CHT II-Fertigsäule von BioRAD) wurde mit mindestens 5 SV Puffer F (20 mM Tris-HCl, pH 7,0; 2 M NaCl; 20% Isopropanol) äquilibriert und anschließend der EPO-haltige Butyl-Sepharose-Pool aus 2. (ca. 125 $\mu$g EPO) bei einem Fluß von 0,5 ml/min aufgezogen.

Die Säule wurde mit mindestens 5 SV Puffer G (20 mM Tris-HCl, pH 7,0; 2 M NaCl) bei 0,5 ml/min gewaschen. Der Wascherfolg wurde über die Messung des Proteingehalts bei OD280 verfolgt.

Die Elution von EPO erfolgte mit Puffer H (10 mM Na-Phosphat, pH 7,0; 80 mM NaCl) bei einem Fluß von 0,5 ml/min. Die Elutionslösung wurde in 1-2 ml Fraktionen gesammelt.

Der EPO-Gehalt der Fraktionen, der Waschlösungen und des Durchlaufs wurden über RP-HPLC durch Auftrag eines Aliquots auf eine POROS R2/H-Säule bestimmt.

EPO-haltige Fraktionen der Elution (3-6 ml) wurden gepoolt. Die Ausbeute der Hydroxyapatit-Säule betrug ca. 80 $\mu$g EPO (entspricht ca. 60%). Im Allgemeinen betrug die Ausbeute der Hydroxyapatit-Säule zwischen 50-65% vom Auftrag der Butyl-Sepharose-Pools.

4. Aufkonzentrierung

Die gepoolten EPO-Fraktionen aus dem Hydroxyapatit-Schritt wurden in Zentrifugationseinheiten mit einer Ausschlußgröße von 10 kD (z.B. Microsep von Filtron) auf eine Konzentration von 0,1-0,5 mg/ml aufkonzentriert, mit 0,01 % Tween 20 versetzt und in Aliquots bei -20°C gelagert.

Ausbeuteschema:

|  | EPO ($\mu$g) | Ausbeute (%) |
|---|---|---|
| Ausgang | 245 | 100 |
| Blue-Sepharose | 175 | 70 |
| Butyl-Sepharose-Säule | 130 | 53 |
| Hydroxyapatit-Säule | 80 | 33 |
| Aufkonzentrierung | 60 | 25 |

Die Reinheit des isolierten EPO war etwa > 90%, in der Regel sogar > 95%.

Zur Erhöhung der EPO-Ausbeute wurde auch die Methode 2 verwendet, bei der der Butyl-Sepharose-Schritt fehlte. Vor allem bei Zellkulturüberständen ohne oder mit 1 % (v/v) FKS-Zusatz ist diese Methode anwendbar und liefert isoliertes EPO von ungefähr gleicher Reinheit (90-95%). Die Anwesenheit von 5 mM $CaCl_2$ im Äquilibrierungspuffer (Puffer F) für die Hydroxyapatit-Säule führte bei dieser Methode zu einer verbesserten Bindung und damit auch zu einem verbessert reproduzierbaren Elutionsverhalten von EPO beim Hydroxyapatit-Schritt. Deshalb wurde bei Methode 2 bei prinzipiell gleichem Ablauf wie Methode 1 mit folgenden Puffern durchgeführt:

1. Blue-Sepharose-Säule:

| Äquilibrierpuffer (Puffer A): | 20 mM Tris-HCl, pH 7,0; 5 mM $CaCl_2$; 100 mM NaCl |
|---|---|
| Waschpuffer 1 (Puffer B): | 20 mM Tris-HCl, pH 7,0; 5 mM $CaCl_2$; 250 mM NaCl |
| Waschpuffer 2 (Puffer C): | 20 mM Tris-HCl, pH 7,0; 5 mM $CaCl_2$, 250 mM NaCl |
| Elutionspuffer (Puffer D): | 100 mM Tris-HCl, pH 7,0; 5 mM $CaCl_2$; 2 M NaCl |

2. Hydroxyapatit-Säule

| Äquilibrierpuffer (Puffer F): | 50 mM Tris-HCl, pH 7,0; 5 mM $CaCl_2$; 1 M NaCl |
|---|---|
| Waschpuffer (Puffer G): | 10 mM Tris-HCl, pH 7,0: 5 mM $CaCl_2$; 80 mM NaCl |
| Elutionspuffer (Puffer H): | 10 mM Na-Phosphat, pH 7,0; 0,5 mM $CaCl_2$; 80 mM NaCl |

Ausbeuteschema:

|  | EPO ($\mu$g) | Ausbeute (%) |
|---|---|---|
| Ausgang | 600 | 100 |
| Blue-Sepharose | 450 | 75 |
| Hydroxyapatit-Säule | 335 | 55 |
| Aufkonzentrierung | 310 | 52 |

Der Zusatz von 5 mM CaCl$_2$ in die Puffer C bis G bei Methode 1 führte ebenfalls zu einer besseren Bindung und definierteren Elution von der Hydroxyapatit-Säule.

Alternativ bzw. zusätzlich können für die Aufreinigung von EPO noch folgende Schritte verwendet werden:

- RP-HPLC z.B. mit Vydac C4-Material
- DEAE-Sepharose ff Chromatographie
- Diafiltration

**Beispiel 2: Aufreinigung von EPO aus Kulturüberständen unter Erhalt der Isoformen 1 - 8 (Vergleich)**

**1. Ausgangsmaterial**

EPO aus Säugerzellen, z.B. CHO- oder humanen Zellen, wurde nach einem Repeated-Batch-Verfahren fermentiert. Ein 1000l Fermenter wurde mit einer Vorkultur angeimpft und nach ca. 3 bis 5 Tagen der Fermenterinhalt geerntet. Nach der Ernte wurden die Zellen aus der Fermentationsbrühe durch Abzentrifugieren entfernt. Der zellfreie Kulturüberstand wird mit 1 mol/l Essigsäure auf pH 5.0 - 5.2 eingestellt und bei 1 - 9 °C filtriert.

**2. Blue-Sepharose-Chromatographie**

Eine Chromatographiesäule (Amicon P440 x 500, Amicon, GB) wurde mit 60 - 80 l Blue Sepharose gefüllt und mit 0,5 N NaOH regeneriert. Anschließend wurde die Säule mit ca. 3 Säulenvolumina (SV) Acetatpuffer äquilibriert.

Der zellfreie, auf pH 5 eingestellte Kulturüberstand wurde bei einer Temperatur von 10 $\pm$ 5 °C und einer Flußrate von 800 - 1400 ml/min auf die Säule aufgezogen. Die Säule wurde bei gleicher Flußrate und 5 $\pm$ 4 °C mit ca. 1 SV Waschpuffer 1 nachgewaschen. Dann folgten ca. 2 SV Waschpuffer 2. Anschließend wurde die Säule mit ca. 3 SV Elutionspuffer eluiert. Der gesamte Proteinpeak wurde gesammelt (ca. 30 - 60 l), mit HCl auf pH 6.9 eingestellt und bis zur Weiterverarbeitung bei 5 $\pm$ 4°C gelagert. Bei diesem Chromatograhieschritt wurde die Produktlösung konzentriert und eine Reinheit von ca. 40 - 50 % erreicht.

| | |
|---|---|
| Äquilibrierpuffer: | 20 mM Na-Acetat, 5 mM CaCl$_2$, 0,1 M NaCl, pH 5.0 $\pm$ 0.2 |
| Waschpuffer 1: | 20 mM Na-Acetat, 5 mM CaCl$_2$, 0,25 M NaCl, pH 5.0 $\pm$ 0.2 |
| Waschpuffer 2: | 20 mM Tris HCl, 5 mM CaCl$_2$, pH 6.5 $\pm$ 0.3 |
| Elutionspuffer: | 100 mM Tris HCl, 5 mM CaCl$_2$, 1 M NaCl, pH 9.0 $\pm$ 0.2 |

**3. Butyl-Toyopearl-Chromatographie (Hydrophobe Chromatographie)**

Eine Chromatographiesäule (Pharmacia BPG 300/500) wurde mit 30 - 40 l Butyl-Toyopearl gefüllt und mit 4 M Guanidin-HCl und 0,5 N NaOH regeneriert. Anschließend wurde die Säule mit mindestens 3 SV Äquilibrierpuffer äquilibriert.

Das Eluat der Blue Sepharose-Säule wurde auf 10 % Isopropanol eingestellt und bei einer Temperatur von 27 $\pm$ 2 °C und einer Flußrate von 800 - 1200 ml/min auf die Säule aufgezogen. Die Säule wurde bei gleicher Temperatur und Flußrate mit ca. 1 SV Äquilibrierpuffer und dann mit ca. 2 SV Waschpuffer nachgewaschen. Anschließend wurde sie mit ca. 3 SV Elutionspuffer eluiert. Der gesamte Proteinpeak wird gesammelt (ca. 10 - 18 l), sofort mit Verdünnungspuffer um den Faktor 3 verdünnt und bis zur Weiterverarbeitung bei 15°C gelagert. Bei dieser Chromatographie wurde eine Reinheit von ca. 90 % erreicht.

| | |
|---|---|
| Äquilibrierpuffer: | 20 mM Tris-HCl, 5 mM CaCl$_2$, 0,75 M NaCl, 10 % Isopropanol, pH 6.9 $\pm$ 0.2 |
| Waschpuffer: | 20 mM Tris-HCl, 5 mM CaCl2, 0,75 M NaCl, 19 % Isopropanol, pH 6.9 $\pm$ 0.2 |

(fortgesetzt)

| Elutionspuffer: | 20 mM Tris-HCl, 5 mM CaCl$_2$, 0,75 M NaCl, 27 % Isopropanol, pH 6.9 $\pm$ 0.2 |
|---|---|
| Verdünnungspuffer: | 20 mM Tris-HCl, 5 mM CaCl$_2$, pH 6.9 $\pm$ 0.2 |

### 4. Hydroxyapatit Ultrogel-Chromatographie

Eine Chromatographiesäule (Amicon P440 x 500 oder Äquivalent) wurde mit 30 - 40 l Hydroxyapatit Ultrogel gepackt und mit 0,5 N NaOH regeneriert. Anschließend wurde die Säule mit mindestens 4 SV Äquilibrierpuffer äquilibriert.

Das Eluat der Butyl-Toyopearl Säule wurde bei einer Temperatur von ca. 15°C und einer Flußrate von 500 - 1200 ml/min auf die Säule aufgezogen. Die Säule wurde bei gleicher Temperatur und Flußrate mit ca. 1 SV Äquilibrierpuffer und dann mit ca. 2 SV Waschpuffer nachgewaschen. Anschließend wurde sie mit ca. 3 SV Elutionspuffer eluiert. Der gesamte Proteinpeak wurde gesammelt (ca. 10 - 18 l) und bis zur Weiterverarbeitung bei 15°C gelagert. Bei dieser Chromatographie wurde eine Reinheit von mehr als 95 % erreicht.

| Äquilibrierpuffer: | 20 mM Tris-HCl, 5 mM CaCl$_2$, 0,25 M NaCl, 9 % Isopropanol, pH 6.9 $\pm$ 0.2 |
|---|---|
| Waschpuffer: | 10 mM Tris-HCl, 5 mM CaCl$_2$, pH 6.8 $\pm$ 0.2 |
| Elutionspuffer: | 10 mM Tris-HCl, 10 mM K-Phosphat, 0,5 mM CaCl$_2$, pH 6.8 $\pm$ 0.2 |

### 5. Reversed Phase HPLC (RP-HPLC)

Eine präparative HPLC wurde mit einer Merck Prepbar 100-Trennanlage (oder Äquivalent) bei einer Temperatur von 22 $\pm$ 4 °C durchgeführt. Die Trennsäule (100 mm x 400 mm, 3,2 l) war mit Vydac C4-Material gepackt. Vor dem Einsatz wurde die Säule durch mehrfaches Anlegen eines Gradienten von Puffer A nach 100 % Lösungsmittel regeneriert und anschließend mit Puffer A äquilibriert.

Das Eluat der Hydroxyapatit-Säule wurde mit Trifluoressigsäure auf ca. pH 2.5 angesäuert und sterilfiltriert. Anschließend wurde es bei einer Temperatur von 22 $\pm$ 4 °C und einer Flußrate von 250 - 310 ml/min auf die Säule aufgezogen. Die Säule wurde bei gleicher Temperatur und Flußrate mit einem linearen Gradient von Puffer A nach Puffer B eluiert. Der Elutionspeak wurde in Fraktionen aufgefangen. Durch Vorlegen von 4 Volumina HPLC-Verdünnungspuffer wurde das Eluat sofort neutralisiert.

Fraktionen, die bei der analytischen HPLC eine Reinheit von mindestens 99 % aufweisen, wurden vereinigt (Poolvolumen ca. 4 - 6 l). Bei dieser Chromatographie wurden Spurenverunreinigungen abgetrennt und eine Reinheit von besser 99 % erreicht.

| Puffer A: | 0,1 % Trifluoressigsäure in Wasser |
|---|---|
| Puffer B: | 80 % Acetonitril, 0,1 % Trifluoressigsäure in Wasser |
| HPLC-Verdünnungspuffer: | 10 mM Na/K-Phosphat, pH 7.5 $\pm$ 0.2 |

### 6. DEAE-Sepharose ff Chromatographie

Eine Chromatographiesäule (Amicon P90 x 250 oder Äquivalent) wurde mit 100 - 200 ml Gel pro g EPO im Auftrag gefüllt und mit 0,5 N NaOH regeneriert. Anschließend wurde die Säule zunächst mit 100 mM Na/K-Phosphatpuffer, pH 7.5 und dann mit mindestens 12 SV Äquilibrierpuffer äquilibriert.

Das Eluat der HPLC-Säule wurde bei einer Temperatur von 5 $\pm$ 4 °C und einer Flußrate von ca. 150 ml/min auf die Säule aufgezogen. Die Säule wurde bei gleicher Temperatur und Flußrate mit mindestens 5 SV Äquilibrierpuffer und dann mit ca. 10 SV Waschpuffer gewaschen. Anschließend wurde sie erneut mit ca. 10 SV Äquilibrierpuffer gewaschen und dann mit ca. 7 SV Elutionspuffer eluiert. Der gesamte Proteinpeak wurde gesammelt (ca. 2 - 5 l), sterilfiltriert und abgefüllt.

Bei dieser Chromatographie wurde das Lösungsmittel aus dem HPLC-Schritt abgetrennt und Spurenverunreinigungen entfernt. Die Reinheit ist besser 99%.

| Äquilibrierpuffer: | 10 mM Na/K-Phosphat, pH 7.5 $\pm$ 0.2 |
|---|---|
| Waschpuffer: | 30 mM Na-Acetat, pH 4.5 $\pm$ 0.1 |
| Elutionspuffer: | 10 mM Na/K-Phosphat, 80 mM NaCl pH 7.5 $\pm$ 0.2 |

**Beispiel 3: Aufreinigung von EPO aus Kulturüberständen unter Erhalt der Isoformen 1 - 4 (Erfindung)**

**1. Ausgangsmaterial**

EPO aus Säugerzellen, z.B. CHO- oder humanen Zellen, wurde nach dem Repeated-Batch-Verfahren fermentiert. Ein 10 l Fermenter wurde mit einer Vorkultur angeimpft und nach ca. 5 Tagen der Fermenterinhalt geerntet. Nach der Ernte wurden die Zellen aus der Fermentationsbrühe durch Abzentrifugieren entfernt. Der zellfreie Kulturüberstand wurde mit 1 mol/l Essigsäure auf pH 5.0 - 5.2 eingestellt und bei 1 - 9 °C filtriert.

**2. Blue-Sepharose-Chromatographie**

Eine geeignete Chromatographiesäule wurde mit 150 - 250 ml Blue-Sepharose gefüllt und mit 0,5 N NaOH regeneriert. Anschließend wurde die Säule mit ca. 3 Säulenvolumina (SV) Acetatpuffer äquilibriert.

Der zellfreie, auf pH 5 eingestellte Kulturüberstand wurde bei einer Temperatur von 10 ± 5 °C und einer Flußrate von 1 - 2 SV/h auf die Säule aufgezogen. Die Säule wurde bei gleicher Flußrate und 5 ± 4 °C mit ca. 1 SV Waschpuffer 1 nachgewaschen. Dann folgten ca. 2 SV Waschpuffer 2. Anschließend wurde die Säule mit ca. 3 - 6 SV Elutionspuffer eluiert. Der Proteinpeak wurde fraktioniert gesammelt. Nach CE-Analytik wurden geeignete Fraktionen gepoolt, mit HCl auf pH 6.9 eingestellt und bis zur Weiterverarbeitung bei 5 ± 4 °C gelagert. Bei diesem Chromatograhieschritt wurde die Produktlösung konzentriert und Verunreinigungen, sowie basische Isoformen abgetrennt.

| | |
|---|---|
| Äquilibrierpuffer: | 20 mM Na-Acetat, 5 mM $CaCl_2$, 0,1 M NaCl, pH 5.0 ± 0.2 |
| Waschpuffer 1: | 20 mM Na-Acetat, 5 mM $CaCl_2$, 0,25 M NaCl, pH 5.0 ± 0.2 |
| Waschpuffer 2: | 20 mM Tris HCl, 5 mM $CaCl_2$, pH 6.5 ± 0.3 |
| Elutionspuffer: | 50 mM Tris HCl, 5 mM $CaCl_2$, 0,25 M NaCl, pH 8.0 ± 0.2 |

**3. Butyl-Toyopearl-Chromatographie (hydrophobe Chromatographie)**

Eine geeignete Chromatographiesäule wurde mit 200 - 300 ml Butyl-Toyopearl gefüllt und mit 4 M Guanidin-HCl.und 0,5 N NaOH regeneriert. Anschließend wurde die Säule mit mindestens 3 SV Äquilibrierpuffer äquilibriert.

Das Eluat der Blue Sepharose-Säule wurde auf 10 % Isopropanol eingestellt und bei einer Temperatur von 27 ± 2 °C und einer Flußrate von 1 - 2 SV/h auf die Säule aufgezogen. Die Säule wurde bei gleicher Temperatur und Flußrate mit ca. 1 SV Äquilibrierpuffer und dann mit ca. 2 SV Waschpuffer nachgewaschen. Anschließend wurde sie mit ca. 5 - 10 SV Elutionspuffer eluiert. Von dem Proteinpeak wurden Fraktionen gesammelt und sofort mit Verdünnungspuffer um den Faktor 3 verdünnt. Nach CE-Analytik wurden geeignete Fraktionen gepoolt und bis zur Weiterverarbeitung bei 15 °C gelagert. Bei dieser Chromatographie wurden weitere basische Isoformen abgetrennt und eine Reinheit von > 80 % erreicht.

| | |
|---|---|
| Äquilibrierpuffer: | 20 mM Tris-HCl, 5 mM $CaCl_2$, 0,2 M NaCl, 10 % Isopropanol, pH 6.9 ± 0.2 |
| Waschpuffer: | 20 mM Tris-HCl, 5 mM $CaCl_2$, 0,2 M NaCl, 17 % Isopropanol, pH 6.9 ± 0.2 |
| Elutionspuffer: | 20 mM Tris-HCl, 5 mM $CaCl_2$, 0,2 M NaCl, 23 % Isopropanol, pH 6.9 ± 0.2 |
| Verdünnungspuffer: | 20 mM Tris-HCl, 5 mM $CaCl_2$, pH 6.9 ± 0.2 |

**4. Hydroxyapatit Ultrogel-Chromatographie**

Eine geeignete Chromatographiesäule wurde mit 150 - 200 ml Hydroxyapatit Ultrogel gepackt und mit 0,5 N NaOH regeneriert. Anschließend wurde die Säule mit mindestens 4 SV Äquilibrierpuffer äquilibriert.

Das Eluat der Butyl-Toyopearl Säule wurde bei einer Temperatur von ca. 15°C und einer Flußrate von 1 - 2 SV/h auf die Säule aufgezogen. Die Säule wurde bei gleicher Temperatur und Flußrate mit ca. 1 SV Äquilibrierpuffer und dann mit ca. 2 SV Waschpuffer nachgewaschen. Anschließend wurde sie mit ca. 3 SV Elutionspuffer eluiert. Der gesamte Proteinpeak wurde gesammelt und bis zur Weiterverarbeitung bei 15°C gelagert. Bei dieser Chromatographie wurde eine Reinheit von besser 95 % erreicht.

| | |
|---|---|
| Äquilibrierpuffer: | 20 mM Tris-HCl, 5 mM $CaCl_2$, 0,06 M NaCl, 7,5 % Isopropanol, pH 6.9 ± 0.2 |
| Waschpuffer: | 10 mM Tris-HCl, 5 mM $CaCl_2$, pH 6.8 ± 0.2 |
| Elutionspuffer: | 10 mM Tris-HCl, 10 mM K-Phosphat, 0,5 mM $CaCl_2$, pH 6.8 ± 0.2 |

## 5. Reversed Phase HPLC (RP-HPLC)

Eine semipräparative HPLC wurde mit einer Vydac C4 - Trennsäule (20 mm x 250 mm, ca. 80 ml) bei einer Temperatur von 22 ± 4 °C durchgeführt. Vor dem Einsatz wurde die Säule durch mehrfaches Anlegen eines Gradienten von Puffer A nach 100 % Lösungsmittel regeneriert und anschließend mit Puffer A äquilibriert.

Das Eluat der Hydroxyapatit-Säule wurde bei einer Temperatur von 22 ± 4°C und einer Flußrate von 8 - 15 ml/min auf die Säule aufgezogen. Die Säule wurde bei gleicher Temperatur und Flußrate mit einem linearen Gradienten von Puffer A nach Puffer B gemäß folgendem HPLC-Protokoll eluiert. Der Elutionspeak wurde in Fraktionen aufgefangen. Durch Vorlegen von 4 Volumina HPLC-Verdünnungspuffer wurde das Eluat sofort verdünnt.

H PLC-Protokoll:

| Zeit (min) | Schritt | % Puffer A (v/v) | % Puffer B (v/v) |
|---|---|---|---|
| | Start | | |
| 0,0 | | 100 | 0 |
| | Wasch 1 | | |
| 10,0 | | 100 | 0 |
| | Wasch 2 | | |
| 20,0 | | 50 | 50 |
| | Wasch 3 und Elution (*) | | |
| 160,0 | | 0 | 100 |
| | Nachwasch | | |
| 170,0 | | 0 | 100 |
| | Zurückstellen auf Ausgangsbedingungen | | |
| 171,0 | | 100 | 0 |
| (*) Wasch 3 und Elutionsbedingungen: Gradient von 50% Puffer B nach 100% Puffer B in 50 bis 200 min, bevorzugt 140 min. | | | |

Fraktionen, die bei der analytischen HPLC eine Reinheit von mindestens 99% aufweisen und nach CE-Analytik geeignet sind, wurden vereinigt. Bei dieser Chromatographie wurden Spurenverunreinigungen sowie Reste basischer Isoformen abgetrennt und eine Reinheit von besser 99 % erreicht.

Puffer A:                         10 mM Na/K-Phosphat, pH 7.0 ± 0.2
Puffer B:                         10 mM Na/K-Phosphat, 80 % Acetonitril, pH 7.0 ± 0.2
HPLC-Verdünnungspuffer:   10 mM Na/K-Phosphat, 100 mM NaCl, pH 7.5 ± 0.2

## 6. Diafiltration

Eine Diafiltrationsapparatur geeigneter Größe wurde mit einer 10 kD Kassette bestückt und mit 1 N NaOH regeneriert. Anschließend wurde die Apparatur mit Bulkpuffer laugefrei gespült.

Das Eluat der HPLC-Säule wurde bei einer Temperatur von 5 ± 4 °C konzentriert und gegen 10 Volumina an Bulkpuffer diafiltriert. Die Endkonzentration sollte zwischen 1 - 3 mg/ml betragen.

Dieser Schritt dient zur Entfernung von Lösungsmittelresten aus dem HPLC-Schritt, zur Einstellung der erforderlichen Bulkpufferbedingungen und Produktkonzentration des Bulk-Wirkstoffs.

Bulkpuffer:        10 mM Na/K-Phosphat, 100 mM NaCl, pH 7.5 ± 0.2

**Beispiel 4 Bestimmung der spezifischen Aktivität von EPO in vivo (Bioassay an der normozythämischen Maus)**

Die dosisabhängige Aktivität von EPO auf die Vermehrung und Differenzierung von Erythrozyten-Vorläuferzellen wurde in vivo in Mäusen über den Anstieg der Reticulocyten im Blut nach EPO-Gabe bestimmt.

Hierzu wurden jeweils acht Mäusen verschiedene Dosen der zu analysierenden EPO-Probe und eines EPO-Standards (abgeglichen gegen den EPO-WHO-Standard) parenteral verabreicht. Die Mäuse wurden anschließend unter konstanten definierten Bedingungen gehalten. 4 Tage nach EPO-Gabe wurde den Mäusen Blut entnommen und die Reticulocyten mit Acridinorange angefärbt. Die Bestimmung der Reticulocytenzahl pro 30 000 Erythrocyten erfolgte mikrofluorimetrisch im Durchflußcytometer durch Analyse des Rotfluoreszenz-Histogramms.

Die Berechnung der biologischen Aktivität erfolgte aus den Werten für die Reticulocytenzahlen der Probe und des Standards bei unterschiedlichen Dosen nach dem von Linder beschriebenen Verfahren der paarweisen Gehaltsbestimmung mit parallelen Geraden (Linder, Planen und Auswerten von Versuchen, 3. Auflage, 1969, Birkenhäuser Verlag, Basel).

Bei der Aufreinigung von EPO aus Kulturüberständen von in serumfreiem Medium kultivierten CHO-Zellen wurden die EPO-Präparate CHO 1, CHO 2 bzw. CHO 3 erhalten, welche eine biologische Aktivität von 248 000 (CHO 1), 225 000 (CHO 2) bzw. 186 000 IU/mg (CHO 3) hatten. In vier Ansätzen, bei denen EPO aus Kulturüberständen von humanen Zellen aufgereinigt wurde, erhielt man Produkte mit spezifischen Aktivitäten von 220 000 (HeLa 1), 198 000 (HeLa 2), 204 000 (HeLa 3), 176 000 (HeLa 4) bzw. 100 000 IU/mg (HeLa 5). Die Korrelation der Werte für die biologische Aktivität mit Parametern der Zuckerstruktur ist in Beispiel 11 angegeben.

**Beispiel 5 Bestimmung des Gehalts an Sialinsäureresten**

Die Bestimmung des Sialinsäuregehaltes erfolgte chromatographisch über HPAEC-PAD (High pH Anion Exchange Chromatography mit Pulsed Amperometric Detection) auf einem Dionex System nach enzymatischer Abspaltung der Sialinsäuren mit Neuraminidase aus Arthrobacter ureafaciens (A. ureaf., Boehringer Mannheim).

Ansätze mit je 22 µg EPO aus verschiedenen Präparationen aus CHO- und humanen Zellinien (z.B. HeLa S3) wurden auf eine EPO-Konzentration von 0,2 mg/ml in 5 mM Na-Phosphat-Puffer, pH 7,2 eingestellt. Je eine Hälfte jedes Ansatzes wurde für die genaue Bestimmung der EPO Menge über RP-HPLC verwendet. Zur 2. Hälfte der Ansätze wurden je 5 mM U Neuraminidase von A. ureaf. gegeben und über Nacht (ca. 18 h) bei 37°C inkubiert. Anschließend wurden die Verdauansätze halbiert, 20-fach auf 500 µl mit $H_2O$ verdünnt und 50 µl davon (entspricht ca. 27 pmol EPO) auf das Dionex System aufgetragen. Hierzu wurden folgende Chromatographie-Parameter verwendet:

| Säule: | CarboPac PA 100 |
|--------|-----------------|
| Fluß: | 1,0 ml/min |
| Detektorempfindlichkeit: | 300 nA |

| Gradient: | t (min) | % Puffer B |
|-----------|---------|------------|
| | 0 | 17 |
| | 7 | 17 |
| | 9 | 100 |
| | 12 | 100 |
| | 13 | 0 |
| | 20 | 0 |

| Puffer A: | 0,1 M NaOH |
|-----------|-------------|
| Puffer B: | 0,1 M NaOH; 0,5 M Na-Acetat |

Die Menge an Sialinsäuren in der aufgetragenen Probe wurde mit Hilfe einer Eichgerade, die aus Werten eines mitgeführten Sialinsäurestandards (Boehringer Mannheim) erhalten wurde, ermittelt. Der Sialinsäuregehalt (mol Sialinsäure/mol EPO) wurde aus dem Ergebnis der Sialinsäurebestimmung (Dionex System) und der Bestimmung der eingesetzten EPO-Menge über RP-HPLC berechnet.

Das EPO aus CHO-Zellen hatte einen mittleren Gehalt von 12,9 mol (CHO 1), 11,8 (CHO 2) bzw. 11,7 mol (CHO 3) Sialinsäure pro mol EPO. Die aus humanen Zellen stammenden EPO-Präparationen hatten einen Anteil von 13,1 mol (HeLa 1), 13,2 mol (HeLa 2), 13,3 mol (HeLa 3), 11,6 mol (HeLa 4) bzw. 10,8 mol (HeLa 5) Sialinsäure pro mol EPO (vgl. auch Beispiel 11).

**Beispiel 6 Bestimmung der Anteile an bi-, tri- und tetraantennären Kohlenhydratstrukturen**

Die Analytik der N-gebundenen Kohlenhydratstrukturen erfolgte chromatographisch über HPAEC-PAD auf einem Dionex System. Die asialo Oligosaccharide von EPO-Präparationen aus CHO- und humanen Zellinien (z.B. HeLa S3) wurden durch enzymatische Abspaltung mit N-Glycosidase F (Boehringer Mannheim) und Neuraminidase aus A.urea.f. (Boehringer Mannheim) gewonnen.

10 bzw. 30 $\mu$g EPO je Ansatz wurden mittels MicroCon-Ultrazentrifugationseinheiten (Amicon, Ausschlußgröße 10 kD) entsalzt und mit 10 mM Na-Phosphat-Puffer, pH 7,2 auf eine Konzentration von 0,2 bzw. 0,3 mg/ml eingestellt. Anschließend wurde jeder Ansatz mit 1 U N-Glycosidase F und 10 mU Neuraminidase versetzt und über Nacht (ca 18 h) bei 37°C inkubiert. Zur Abtrennung des EPO-Polypeptidanteils von den abgespaltenen Oligosacchariden wurden die Ansätze nach der Inkubation durch Ultrafree-Zentrifugationseinheiten (Millipore, Ausschlußgröße 10 kD) zentrifugiert und das Ultrafree-Device zweimal mit 20 $\mu$l $H_2O$ nachgewaschen. Die im Durchlauf enthaltenen Oligosaccharide wurden mit $H_2O$ auf 150 $\mu$l aufgefüllt und 100 $\mu$l davon auf dem Dionex System analysiert. Hierzu wurden folgende Chromatographie-Parameter verwendet:

| Säule: | CarboPac PA 100 |
|---|---|
| Fluß: | 1,0 ml/min |
| Detektorempfindlichkeit: | 300 nA |

| Gradient: | t (min) | % Puffer B |
|---|---|---|
| | 0 | 0 |
| | 2 | 0 |
| | 60 | 10 |
| | 62 | 100 |
| | 67 | 100 |

| t (min) | % Puffer B |
|---|---|
| 69 | 0 |
| 80 | 0 |

| Puffer A: | 0,1 M NaOH |
|---|---|
| Puffer B: | 0,1 M NaOH; 0,5 M Na-Acetat |

Die Identifikation der Peaks in einem Chromatogramm von N-Zuckern des komplexen Typs erfolgte durch Standard-Oligosaccharide (Oxford Glyco Systems) und wurde durch den enzymatischen Verdau der Oligosaccharide von EPO mit dem Enzym Endo-$\beta$-Galactosidase bzw. Fucosidase und anschließender Analytik auf dem Dionex System verifiziert. Die Berechnung der prozentualen Anteile an bi-, tri- und tetraantennären Strukturen erfolgte über die Flächen der Peaks, die die entsprechende N-Zuckerstruktur repräsentieren, bezogen auf die Gesamtpeakfläche (Summe der Peakflächen von bi-, tri- und tetraantennären Strukturen).

Das aus CHO-Zellen stammende EPO hatte einen Anteil von 4,2% biantennären Kohlenhydratstrukturen, 22,3% triantennären Kohlenhydratstrukturen und 73,5% tetraantennären Kohlenhydratstrukturen (CHO3) bzw. einen Anteil von 86,7% tetraantennären Kohlenhydratstrukturen in Präparat CHO 1 und 78,6% in CHO 2. Bei den Präparationen von EPO aus humanen Zellinien wurden Anteile an biantennären/triantennären/tetraantennären Strukturen für HeLa 1 von 5,8/8,8/85,4 %, für HeLa 2 von 5,1/12,7/82,2%, für HeLa 3 von 4,1/17,7/78,2%, für HeLa 4 von 10,1/19,2/70,6% und für HeLa 5 von 12,6/25,4/62% erhalten (vgl. auch Beispiel 11).

**Beispiel 7 Bestimmung des mittleren Gehalts an N-Acetyl-Lactosamin-Einheiten und des mittleren Anteils an zusätzlichen N-Acetyl-Lactosamin-Einheiten (Repeats)**

Die Gesamtzahl an N-Acetyl-Lactosamin-Einheiten in den N-gebundenen Kohlenhydratstrukturen von EPO (d.h. in den Core-Kohlenhydratstrukturen plus Repeats) wurde aus den Peakflächen der Chromatogramme der Experimente von Beispiel 6 berechnet.

Die Berechnung der Anzahl des mittleren Gehalts (n) von N-Acetyl-Lactosamin-Einheiten pro Kohlenhydratkette war wie folgt.

$$n = \Sigma \ \% \ (bi)x2 + \ \% \ (tri) \ x3 + \ \% \ (tetra) \ x4 + \ \% \ (tri + 1r)x4 + \ \% \ (tetra + 1r) \ x5 + \ \% \ (tri + 2r)x5 + \% \ (tetra + 2r)x6$$

wobei

% (bi)=  prozentualer Anteil biantennärer Strukturen bezogen auf die Gesamtmenge (100%) von N-gebundenen Kohlenhydratstrukturen

% (tri) =  prozentualer Anteil triantennärer Strukturen ohne zusätzliche N-Acetyl-Lactosamin-Einheit

% (tetra) =  prozentualer Anteil tetraantennärer Strukturen ohne zusätzliche N-Acetyl-Lactosamin-Einheit

% (tri + 1r) =  prozentualer Anteil triantennärer Strukturen mit 1 zusätzlichen N-Acetyl-Lactosamin-Einheit

% (tetra + 1r) =  prozentualer Anteil tetraantennärer Strukturen mit 1 zusätzlichen N-Acetyl-Lactosamin-Einheit

% (tri + 2r) =  prozentualer Anteil triantennärer Strukturen mit 2 zusätzlichen N-Acetyl-Lactosamin-Einheiten

% (tetra + 2r) =  prozentualer Anteil tetraantennärer Strukturen mit 2 zusätzlichen N-Acetyl-Lactosamin-Einheiten.

Bei EPO aus CHO-Zellen wurde eine mittlere Anzahl von 4,3 (CHO 1), 4,4 (CHO 2) bzw. 4,2 (CHO 3) N-Acetyl-Lactosamin-Einheiten pro Kohlenhydratkette gefunden. Bei den EPO-Präparationen aus humanen Zellen wurde eine Anzahl an N-Acetyl-Lactosamin-Einheiten von 4,0 (HeLa 1), 4,0 (HeLa 2), 3,9 (HeLa 3), 3,75 (HeLa 4) bzw. 3,6 (HeLa 5) gefunden (vgl. auch Beispiel 11).

Aufgrund der Tatsache, daß EPO 3 N-gebundene Zuckerstrukturen enthält, ist die Gesamtzahl an N-Acetyl-Lactosamin-Einheiten um das 3-fache größer. Bezogen auf die Gesamtglycosilierung von EPO beträgt die Anzahl der N-Acetyl-Lactosamin-Einheiten bei EPO aus CHO-Zellen deshalb 12,9 (CHO 1), 13,2 (CHO 2) bzw. 12,6 (CHO 3). Bei den EPO-Präparaten aus humanen Zellen waren die entsprechenden Werte 12,0 (HeLa 1), 11,9 (HeLa 2), 11,7 (HeLa 3), 11,25 (HeLa 4) und 10,8 (HeLa 5).

Das Produkt aus Anzahl der N-Acetyl-Lactosamin-Einheiten pro Kohlenhydratstruktur multipliziert mit dem jeweiligen Sialinsäuregehalt ergab bei EPO aus CHO-Zellen Werte von 55,5 (CHO 1), 52 (CHO 2) bzw. 49,3 (CHO 3).

Bei den EPO-Präparaten aus humanen Zellen waren die entsprechenden Werte 52,4 (HeLa 1), 52,5 (HeLa 2), 51,3 (HeLa 3), 43,5 (HeLa 4) und 38,9 (HeLa 5).

Bezogen auf die Gesamtglycosilierung von EPO (3 N-gebundene Kohlenhydratstrukturen) beträgt das Produkt aus Anzahl der N-Acetyl-Lactosamin-Einheiten, multipliziert mit dem jeweiligen Sialinsäuregehalt bei EPO aus CHO-Zellen 166,5 (CHO 1), 156 (CHO 2) bzw. 147,9 (CHO 3). Bei den EPO-Präparaten aus humanen Zellen waren die entsprechenden Werte 157,2 (HeLa 1), 157,5 (HeLa 2), 153,9 (HeLa 3), 130,5 (HeLa 4) und 116,7 (HeLa 5), vgl. auch Beispiel 11.

Ein weiterer wichtiger Parameter ist der Anteil von N-Acetyl-Lactosamin-Einheiten die als sogenannte Repeats an die Core-Kohlenhydratstrukturen gebunden sein können (vgl. z.B. Fig. 1). Der Repeatgehalt wird als prozentualer Anteil der repeathaltigen Kohlenhydratstrukturen bezogen auf die Summe aller N-gebundenen Kohlenhydratstrukturen (bi +tri +tetra = 100%) angegeben.

Dieser Anteil von Repeats kann bei EPO-Präparaten aus CHO-Zellen und aus humanen Zellen unterschiedlich sein. So wurden für die Präparate aus CHO-Zellen Repeatsanteile von 39,6% (CHO 1), 51 % (CHO 2) bzw. 36,8% (CHO 3). Für die Präparate aus humanen Zellen wurden die Repeatanteile von 18% (HeLa 1), 16,5% (HeLa 2), 14,0% (HeLa 3), 12,2% (HeLa 4) und 9,8% (HeLa 5) bestimmt (vgl. Beispiel 11).

**Beispiel 8: Beeinflussung der biologischen Aktivität von EPO durch kontrollierte und bedarfsgerechte Zufütterung**

Die Kultivierungen wurden als wiederholt satzweiser Betrieb mit bedarfsgerechter Zufütterung (Repeated Fed Batch) bei einer Temperatur von 36,5 °C durchgeführt. Dazu wurde in einem gerührten Fermenter (Gesamtarbeitsvolumen: 10 L) serumfreies proteinarmes Kulturmedium vorgelegt und einmalig mit einer Inokulumskultur beimpft. Die Zelldichte nach Beimpfen lag im Bereich $3 \pm 1\text{x}10^5$ lebende Zellen/ml.

Nach einer Wachstumsphase von $144 \pm 24$ Stunden wurde ein Teil der Kulturbrühe geerntet. Der Rest der Kulturbrühe verblieb im Fermenter und stellte das Inokulum für die nächste Wachstumsphase dar; dazu wurde der Fermenter wieder bis zum Arbeitsvolumen mit frischem Medium aufgefüllt.

Der EPO-haltige Kulturüberstand wurde durch Zentrifugation der Fermentationskultur erhalten.

Während der Wachstumsphase wurde der Kultur kontinuierlich Nährstofflösung zugeführt. Dazu wurde ein Vorratsgefäß mit Nährlösung an den Fermenter gekoppelt. Die Nährstofflösung enthielt Aminosäuren, Vitamine, Insulin, Spurenelemente, Salze, Glutamin und Kohlenhydrate. Es wurden 2 Fermentationen wie folgt durchgeführt:

Bei der Fermentation A enthielt die Nährstofflösung als Zucker D-( + )-Glucose und bei der Fermentation B die Zucker D-( + )-Glucose, D-( + )-Galactose und D-( + )-Mannose. Das Masseverhältnis von Glutamin zu den Zuckern betrug bei Fermentation B 1:2,2:3,6:6. Die Konzentration der einzelnen Zucker in der Nährstofflösung lag zwischen 7,2 und 18 g/l.

Die Glutaminkonzentration in der Kultur wurde bei Fermentation B periodisch analysiert und der Verbrauch berechnet. Der momentane Volumenstrom der Nährstofflösung wurde dem Bedarf der Zellen an Nährstoffen angepaßt. Bei Fermentation A wurde die Glutaminkonzentration nicht als Regelgröße verwendet. Die Nährstofflösung bei der Fermentation B enthielt eine Mischung der Zucker D-( + )Glucose, D-( + )Galactose und D-( + )Mannose im Massenverhältnis von 2:3:5. Während der Kultivierung wurde durch entsprechende Zufütterung die Konzentration aller Zucker im Fermenter in einem Bereich von 2 bis 6 g/l gehalten.

Die Zelldichte veränderte sich durch das Wachstum auf mehr als $20\text{x}10^5$ Zellen/ml, typischerweise $30 \pm 10\text{x}10^5$ Zellen/ml zum Erntezeitpunkt. Die Konzentration an EPO betrug zum Erntezeitpunkt typischerweise $40 \pm 10$ mg/l.

Aus geernteten Kulturbrühen wurde die Konzentration an humanem Erythropoietin z.B. durch ELISA bestimmt. Eine prozentuale Verteilung der auftretenden Isoformen dieses Proteins wurden z.B. durch Trennung mittels Kapillarzonenelektrophorese (CZE) bestimmt.

Tabelle 1 zeigt den Vergleich der EPO-Isoformenverteilung zwischen einer Fermentation A mit Zufütterung einer Nährstofflösung mit Glucose und einer Fermentation B mit kontrollierter und bedarfsgerechter Zufütterung einer Nährstofflösung mit Glucose, Mannose und Galactose. Der Anteil der EPO-Isoformen bei Fermentation B wurde als prozentualer Anteil der korrespondierenden Isoformen der Fermentation A berechnet. Letztere wurden auf jeweils 100% normiert. Die Daten belegen, daß die erwünschten höher glykosilierten EPO-Isoformen 2-4 während der Fermentation gegenüber der Fermentation A in maßgeblich höheren Anteil vorliegen.

Tabelle 1:

| Isoformbezeichnung in der CZE | 1 | 2 | 3 | 4 | 5 | 6 | 7 | 8 |
|---|---|---|---|---|---|---|---|---|
| | [%] | [%] | [%] | [%] | [%] | [%] | [%] | [%] |
| Fermentation A: Zufütterung mit Glucose | n.b. | 100 | 100 | 100 | 100 | 100 | 100 | 100 |
| Fermentation B: bedarfsgerechte Zufütterung mit Glucose, Mannose und Galactose | n.b. | 136 | 140 | 115 | 102 | 91 | 76 | 55 |
| n.b.= nicht bestimmbar, da Wert unter Nachweisgrenze | | | | | | | | |

Das bei Zufütterung erhaltene Isoformenmuster war bei vier aufeinanderfolgenden Ernten aus einer Fermentation mit kontrollierter und bedarfsgerechter Zufütterung der Nährstofflösung reproduzierbar.

**Beispiel 9: Beeinflussung der biologischen Aktivität von EPO durch Veränderung der Kultivierungstemperatur**

Die Durchführung erfolgte wie in Beispiel 8 (Fermentation B) beschrieben im Fed-Splitbatch-Verfahren mit kontrollierter und bedarfsgerechter Zufütterung, außer daß die Fermentertemperatur 35,0 °C anstelle von 36,5 °C betrug und die Fermentation im 1000 l Maßstab durchgeführt wurde.

Tabelle 2 zeigt den Vergleich der EPO-Isoformenverteilung zwischen einer Fermentation C bei 36,5 °C und einer Fermentation D bei 35,0 °C jeweils mit kontrollierter Zufütterung einer Nährstofflösung. Der Anteil der EPO-Isoformen bei Fermentation D wurde als prozentualer Anteil der korrespondierenden Isoformen der Fermentation C berechnet. Letztere wurden auf jeweils 100 % normiert. Die Daten belegen, daß die sauren EPO-Isoformen 2 bis 4 durch Absenkung der Temperatur maßgeblich angehoben werden.

Tabelle 2:

| Isoformbezeichnung in der CZE | 1 | 2 | 3 | 4 | 5 | 6 | 7 | 8 |
|---|---|---|---|---|---|---|---|---|
| Relative Isoformenverteilung | [%] | [%] | [%] | [%] | [%] | [%] | [%] | [%] |
| Fermentation C: Temperatur 36,5 °C: | n.b. | 100 | 100 | 100 | 100 | 100 | 100 | 100 |
| Fermentation D: Temperatur 35,0 °C: | n.b. | 131 | 116 | 110 | 94 | 100 | 88 | 86 |
| n.b.= nicht bestimmbar, da Wert unter Nachweisgrenze | | | | | | | | |

## Beispiel 10: Beeinflussung der biologischen Aktivität von EPO durch Veränderung der Kohlenhydratzusammensetzung im Medium

Das im folgenden dargelegte Verfahren belegt die Möglichkeit, die Qualität von humanem Erythropoietin durch Veränderung des Kohlenhydratangebotes im Zufütterungsmedium zu verändern.

Es werden zwei Varianten des oben beschriebenen Verfahrens gezeigt (nachfolgend Fermentation E und Fermentation F genannt), die sich durch die Zusammensetzung der verwendeten Medien unterscheiden.

Für beide Ansätze basierte die Rezeptur des Kulturmediums auf modifiziertem eRDF-Medium. Es wurde kein Serum eingesetzt, sondern rekombinantes Insulin (einziger Proteinzusatz) und weitere Supplemente (z.B. Selenit, Putrescin, Hydrocortison, Eisensulfat), die üblicherweise bei serumfreien bzw. proteinfreien Medien eingesetzt werden.

Die Zufütterungs-Nährlösung basiert ebenfalls auf modifiziertem eRDF-Medium, enthält jedoch nicht die Salze KCl, $Na_2HPO_4$ und NaCl.

Der maßgebliche Unterschied der Fermentationen E und F liegt im Zusatz von verschiedenen Monosacchariden zum Zufütterungsmedium.

Fermentation E:

Es wurde für Fermentation E der üblicherweise eingesetzte Zucker D-( + )-Glucose verwendet. Die Startkonzentration betrug 3 g/l. Durch eine geeignete Zufütterung der glucosehaltigen Nährlösung wurde die Glucosekonzentration in der Kulturbrühe bei 3 ± 0,5 g/l während der gesamten Kultivierung konstant gehalten.

Die Kultivierungsdauer lag typischerweise bei 100 ± 20 h. Die Konzentration an EPO betrug zum Erntezeitpunkt typischerweise 40 ± 10 mg/l.

Fermentation F:

Für die Fermentation F wurden zusätzlich zu D-(+)-Glucose die Zucker D-( + )-Galactose und D-( + )-Mannose im Masseverhältnis von ca. 1 :2:3 für das Zufütterungsmedium eingesetzt. Während der Kultivierung wurde durch entsprechende Zufütterung die Konzentration aller Zucker in einem Bereich zwischen 0,25 g/l und 3,5 g/l gehalten.

Die Kultivierungsdauer für diese Wachstum lag typischerweise bei 100 ± 20 Stunden. Die Konzentration an EPO betrug zum Erntezeitpunkt typischerweise 40 ± 10 mg/l.

Erythropoietin wurde aus den Kulturüberständen aufgereinigt. Das durchgeführte Aufreinigungsverfahren war so ausgelegt (siehe Beispiel 2), daß die Verteilung von relevanten Isoformen des Glykoproteins nicht beeinflußt wurde.

Die Isoformenverteilung des gereinigten Erythropoietin wurde wie zuvor beschrieben bestimmt.

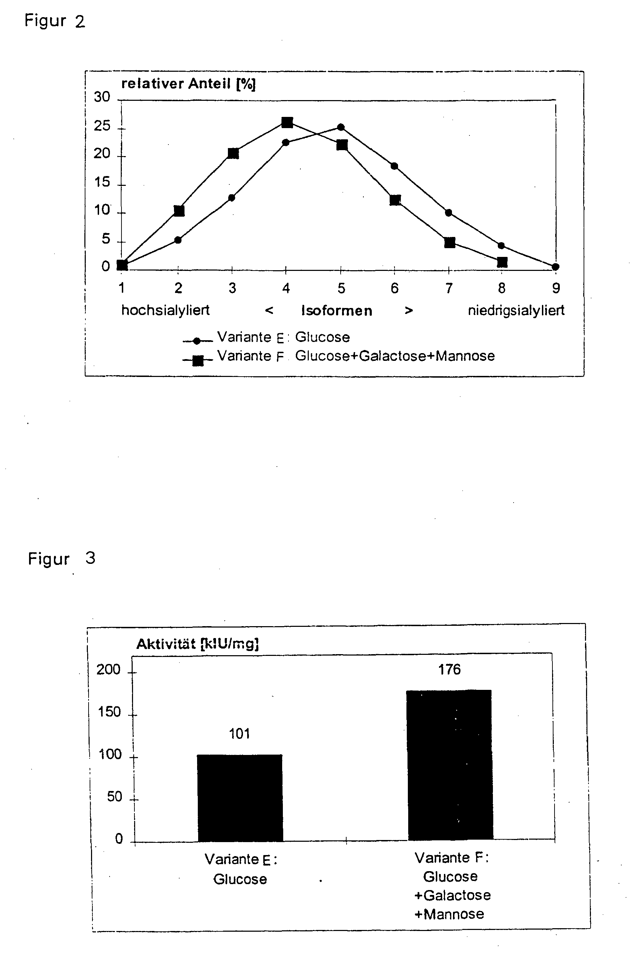

Die Kohlenhydratstrukturen der Isoformen von humanem Erythropoietin und ihre Verteilung unterscheiden sich in den geernteten Kulturüberständen von Fermentation E zu Fermentation F. Die Fermentation E zeigt einen deutlich höheren Anteil der Isoformen 2, 3 und 4 gegenüber Fermentation F. Diese Unterschiede werden durch eine Zufütterung der Monosaccharide Mannose und Galactose bewirkt (vgl. Fig. 2).

Die biologische Aktivität, bestimmt durch den Normo-Maus-Test (Beispiel 4), korreliert mit der Verteilung und den Kohlenhydratstrukturen der EPO-Isoformen (Fig. 3). Die Kohlenhydratstrukturen der aus den Kulturüberständen E und F erhaltenen EPO-Präparationen wurden mit CZE- und HPAEC-Analytik untersucht.

In Tabelle 3 sind die Antennärität (Gehalt an Bi- Tri- und Tetrastrukturen), der Gehalt an N-Acetyl-Lactosamineinheiten (LE), der Sialinsäuregehalt (SA) und das Produkt aus LE und SA der beiden EPO-Präparationen dargestellt.

Tabelle 3

|  | bi [%] | tri [%] | tetra [%] | SA-Gehalt | LE-Gehalt | LExSA |
|---|---|---|---|---|---|---|
|  |  |  |  |  |  |  |
| Fermentation E | 12,6 | 25,4 | 62,0 | 10,8 | 10,8 | 116,7 |
| Fermentation F | 10,1 | 19,2 | 70,6 | 11,6 | 11,25 | 130,5 |

**Beispiel 11: Korrelation der spezifischen Aktivität und Kohlenhydratstrukturen**

In diesem Beispiel sind Untersuchungen zur Abhängigkeit der biologischen Aktivität einzelner EPO-Isoformen von den Kohlenhydratstrukturen zusammengefaßt. Dabei wurden Isoformen (IF) aus verschiedenen EPO-Quellen (verschiedene Chargen von EPO aus CHO-Zellen und humanen Zellen) isoliert und verglichen.

**11.1 Isolierung individueller Isoformen von EPO mittels isoelektrischer Fokussierung (IEF) und Western Blot**

A) Durchführung der IEF-Gelelektrophorese und Elektroblotting auf Nitrozellulose

Zur Isolierung einzelner Isoformen in reiner Form wurde eine EPO-Lösung bestehend aus einem Gemisch mehrerer Isoformen in Ultrafree-Zentrifugationseinheiten entsalzt und aufkonzentriert (5-10 mg/ml). 350-1000 $\mu$g dieser Lösung wurden auf ein IEF-Polyacrylamid-Fertiggel von Serva (Servalyt Precotes, pH 3-5, 300 $\mu$m, 125 x 125 mm) aufgetragen (in 5-10 Bahnen mit 70-100 $\mu$g EPO pro Bahn). Die IEF wurde bei 2500 V für 3,5 h bei 5 °C durchgeführt; anschließend wurde das Gel auf Nitrozellulose geblottet (Naßblot in Tris/Glycin-Puffer mit Methanol, aber <u>ohne</u> SDS für 3 h bei 200 mA). Nach dem Blotvorgang wurde das Gel entfernt und die Nitrozellulosemembran mit Ponceau S angefärbt. Die gefärbten Isoformen wurden ausgeschnitten und mit $H_2O$ oder TBS-Puffer (100 mM Tris, pH 7,4; 150 mM NaCl) wieder vollständig entfärbt.

B) Extraktion der Isoformen von der Membran

Die entfärbten Nitrozellulosestreifen mit den jeweils entsprechenden Isoformen wurden in 2 ml Eppendorfgefäße gegeben (entsprechend 3-4 Bahnen des IEF-Gels), 1,5 ml Aceton zugegeben und die Nitrozellulose durch Vortexen gelöst. Zur optimalen Ausfällung des EPO wurde über Nacht bei -20°C inkubiert. Anschließend wurde das EPO-haltige Präzipitat für 10 min in einer Tischzentrifuge bei 14000 Upm isoliert. Der Niederschlag wurde 2-3 mal mit 1 ml Aceton gewaschen und danach bei Raumtemperatur oder 37°C unter Stickstoffstrom getrocknet. Das EPO-Präzipitat wurde anschließend in 20 mM Na-Phosphat-Puffer, pH 7,2 mit 0,01% Tween 20 gelöst und bis zur weiteren Analyse bei -20°C gelagert.

C) Isolierung von Isoformen aus vorfraktionierten EPO-Lösungen

Die Isolierung einzelner Isoformen wurde wie in A) und B) beschrieben durchgeführt, mit der Einschränkung, daß die verwendeten EPO-Ausgangslösungen nicht 7-8, sondern nur 3-4 Isoformen enthielten. Ausgangsmaterial waren EPO-Fraktionen, die durch eine DE-Chromatographie (Anionenaustauscher) gewonnen wurden. Diese Fraktionen enthielten nur 3-4 Isoformen (z.B. Isoform 6-8 oder Isoform 1-4). Für die Isolierung der "Isoformenpakete" wurde eine geeignete Chromatographiesäule mit 1-2 ml DEAE-Sepharose ff pro 10 mg EPO im Auftrag gefüllt umd mit 0,5 M NaOH regeneriert. Anschließend wurde die Säule zunächst mit 2 SV Neutralisierungspuffer und dann mit mindestens 5 SV Äquilibrierpuffer äquilibriert.

Eine gereinigte EPO-Präparation bestehend aus 8 Isoformen wurde bei einer Temperatur von 5 $\pm$ 4°C und einer Flußrate von bis zu 15 SV/h aufgezogen. Die Säule wurde dann mit 2 bis 3 SV Äquilibrierpuffer gewaschen und anschließend solange mit Waschpuffer gespült, bis der pH-Wert bei 5,0 lag (ca. 5 SV).

Die Elution von verschiedenen Isoformenpaketen wurde durch Erhöhung der NaCl-Konzentration im Elutionspuffer in 10 mM-Stufen beginnend bei 20 mM NaCl erreicht. Die basischen Isoformen binden schwächer an den Ionenaustauscher und eluieren entsprechend bei niedrigen Ionenstärken, die sauren Isoformen eluieren bei höheren NaCl-Konzentrationen bis 70 mM NaCl. Die Menge der bei einer bestimmten NaCl-Konzentration eluierten Isoformen hängt stark vom Ausgangsmaterial und dem Elutionsvolumen ab.

Auf den einzelnen Stufen wurde in der Regel so lange eluiert, bis die OD280 auf etwa 50% des Maximalwertes bei dieser NaCl-Konzentration abgesunken war. Dies entsprach zwischen 15 und 40 SV. Durch zusätzliche Fraktionierung der eluierten Isoformenpakete innerhalb einer NaCl-Konzentration wurde eine weitere Auftrennung der Isoformen erreicht. Die Laufgeschwindigkeit der Säule betrug bis zu 15 SV/h.

| | |
|---|---|
| Neutralisationspuffer: | 100 mM Na/K-Phosphat, pH 7,5 $\pm$ 0,2 |
| Äquilibrierpuffer: | 10 mM Na/K-Phosphat, pH 7,5 $\pm$ 0,2 |
| Waschpuffer: | 30 mM NaAc/HAc, pH 5,0 $\pm$ 0,2 |
| Elutionspuffer: | 10 mM NaAc/HAc, pH 5,0 $\pm$ 0,2, 20 mM NaCl, bzw. Erhöhung der Konzentration in 10 mM Schritten bis 70 mM NaCl |

Aus den so erhaltenen Isoformenpaketen wurden einzelne, reine Isoformen durch Aufreinigung wie in A) und B) beschrieben gewonnen.

Die Nummerierung der aus A-C gewonnenen reinen Isoformen (IF) wurde entsprechend ihres isoelektrischen Punktes (pI) von sauer nach basisch vorgenommen. Isoform 2 (IF2) ist die am stärksten saure isolierte Isoform mit dem niedrigsten pI, Isoform 8 ist die am stärksten basische mit dem höchsten pI. Isoform 2 war die Isoform mit dem niedrigsten pI, die in ausreichenden Mengen aus dem Ausgangsgemisch isoliert werden konnte. Isoform 1 war nur zu 1-2% im Ausgangsgemisch enthalten, so daß für eine vollständige Analyse keine ausreichenden Mengen gewonnen werden konnten.

Es wurden folgende Analysen zur Charakterisierung der reinen Isoformen durchgeführt:

- Bestimmung der Menge und der Ausbeute mittels RP-HPLC

- Bestimmung der Reinheit und Identität durch Kapillarelektrophorese und Isoelektrische Fokussierung

Die Ausbeute an individuellen Isoformen betrug im allgemeinen zwischen 20% und 30% der im Ausgangsgemisch eingesetzten Isoform.

Die Reinheit der Isoformen war in der Regel > 90%, meist sogar > 94%.

11.2 Ergebnisse

Von den gereinigten Isoformen (IF) wurden folgende Daten erhoben:

- Relative Verteilung der N-gebundenen Kohlenhydratstrukturen (Anteil an bi-, tri- und tetraantennären Strukturen an der Gesamtglycosilierung) und des Repeatgehalts

- biologische Aktivität im Normo-Maus-Test

- Sialinsäuregehalt

Diese Bestimmungen wurden im wesentlichen nach den zuvor bereits beschriebenen Methoden durchgeführt.

Der Sialinsäuregehalt isolierter Isoformen wurde nicht für jede einzelne Isoformpräparation separat ermittelt, sondern wurde für jede der Isoformen 2-8 von EPO aus CHO-Zellen bzw. Isoformen 2-6 von EPO aus humanen Zellen beispielhaft an 1-3 Präparationen bestimmt.

Zur Berechnung des Produktes aus Gehalt an N-Acetyl-Lactosamineinheiten (LE-Wert) und dem Sialinsäuregehalt (SA) wurden die gerundeten, ganzzahligen Sialinsäurewerte jeder Isoform verwendet.

Diese gerundeten SA-Werte waren für EPO aus CHO-Zellen und humanen Zellen wie folgt: 14 (IF2), 13 (IF3), 12 (IF4), 11 (IF5), 10 (IF6), 9 (IF7 und 8 (IF8).

Die Tabelle 4 enthält Angaben zur Korrelation zwischen spezifischer Aktivität und Kohlenhydratstrukturen von verschiedenen EPO-Präparaten aus CHO-Zellen (CHO 1, CHO 2 und CHO 3) sowie aus humanen Zellen (HeLa 1 bis 5). Die Tabelle zeigt die Korrelation zwischen der biologischen Aktivität und der durchschnittlichen Gesamtzahl von N-Acetyl-Lactosaminheinheiten (LE) im EPO-Molekül, dem mittleren Sialinsäuregehalt (SA) sowie dem Produkt LE x SA.

Die Tabelle 5 enthält Angaben zur Korrelation zwischen der biologischen Aktivität und der durchschnittlichen Gesamtzahl von N-Acetyl-Lactosaminheinheiten (LE) im EPO-Molekül, dem mittleren Sialinsäuregehalt (SA) sowie dem Produkt LE x SA von isolierten Isoformen einer nicht vorfraktionierten EPO-Charge aus CHO-Zellen.

Die Tabelle 6 enthält einen Vergleich verschiedener Präparationen (A und B) einer Isoform (IF2 bzw. IF5), die aus unterschiedlichen Fraktionen einer vorfraktionierten EPO-Charge aus CHO-Zellen isoliert wurde, d.h. von der Isoform 2 bzw. 5 wurden je 2 Präparationen (A und B) analysiert. Die Vorfraktionierung erfolgte über einen DE-Anionenaustauscher, wie in Beispiel 11.1.C beschrieben. Die beiden Präparationen A und B der Isoform IF2 bzw. IF5 wurden aus unterschiedlichen Fraktionen der DE-Säule isoliert (IF2 aus den Fraktionen 5 und 6 und IF5 aus den Fraktionen 2 und 3). Die Fraktion 5 bzw. 2, aus der IF2/A bzw. IF5/A isoliert wurde, eluierte früher (bei geringerer Salzkonzentration) von der DE-Sepharose-Säule als die Fraktion 6 bzw. 3, aus der IF2/B bzw. IF5/B isoliert wurde. Die Präparationen A und B der Isoform 2 bzw. 5 unterscheiden sich aber nicht in ihrem Verhalten bei der anschließenden isoelektrischer. Fokussierung oder in der Kapillarelektrophorese, d.h. beide Präparationen von IF2- bzw. IF5 haben den gleichen Sialinsäuregehalt. Überraschenderweise wurde jedoch festgestellt, daß die Isoformen aus der Präparation A aufgrund ihres höheren LE-Werts bzw. des höheren Anteils an Repeat-Strukturen eine signifikant höhere biologische Aktivität aufweisen als die entsprechenden Isoformen aus Präparation B. Die in Tabelle 6 beschriebene Abhängigkeit der biologischen Aktivität einer Isoform von der Gesamtzahl der im EPO-Molekül enthaltenen N-Acetyl-Lactosamin-Einheiten bei gleichen Sialinsäuregehalt, wurde nicht nur für Isoform 2 und 5, sondern auch für andere Isoformen beobachtet.

Die Tabelle 7 vergleicht entsprechende Isoformen aus verschiedenen EPO-Quellen (CHO-Zellen oder humane HeLa S3-Zellen). Auch hier wird eine Korrelation der biologischen Aktivität mit dem LE x SA-Wert gefunden.

In allen Tabellen ist somit eine Korrelation des Produkts aus Anzahl der N-Acetyl-Lactosamin-Einheiten (LE) mit dem Sialinsäuregehalt (SA) und der biologischen Aktivität zu erkennen. Ein hoher Wert des Produkts LE X SA ist stets mit einer hohen biologischen Aktivität assoziiert.

Tabelle 4:

| Bezeichnung | tetraant. (in %) | repeat[1] (in%) | LE | SA mol/- mol | LExSA | spezif. Akt. KU/mg |
|---|---|---|---|---|---|---|
| CHO 1 | 86,7 | 39,6 | 12,9 | 12,9 | 166,4 | 248 |
| CHO 2 | 78,6 | 51 | 13,2 | 11,8 | 155,8 | 225 |
| CHO 3 | 73,5 | 42,6 | 12,6 | 11,7 | 147,4 | 186 |
| HeLa 1 | 85,4 | 18,0 | 11,0 | 13,1 | 157,2 | 220 |
| HeLa 2 | 82,2 | 16,5 | 11,9 | 13,2 | 157,1 | 198 |
| HeLa 3 | 78,2 | 14,0 | 11,7 | 13,3 | 155,6 | 204 |
| HeLa 4 | 70,6 | 12,2 | 11,25 | 11,6 | 130,5 | 176 |
| HeLa 5 | 62 | 9,8 | 10,8 | 10,8 | 116,7 | 100 |

LE: N-Acetyl-Lactosamineinheiten
SA: Sialinsäuregehalt des EPO-Präparats
1 : %-Anteil aller Zuckerstrukturen mit zusätzlichen LE-Verlängerungen bezogen auf die Gesamtheit der Zuckerstrukturen (bi + tri + tetra = 100%)

Tabelle 5

|  | tetraant. (in %) | repeat[1] (in %) | LE | SA mol/mol | LExSA | spezif. Akt. KU/mg |
|---|---|---|---|---|---|---|
| IF2 | 98 | 48 | 13,7 | 14 | 191,1 | 400 |
| IF3 | 86 | 43 | 13,1 | 13 | 170,3 | 280 |
| IF4 | 75 | 40 | 12,6 | 12 | 151,2 | 200 |
| IF5 | 64 | 39 | 12,0 | 11 | 132 | 150 |
| IF6 | 56 | 41 | 11,4 | 10 | 114 | 75 |
| IF7 | 42 | 39 | 11,1 | 9,0 | 100 | 40 |
| IF8 | 34 | 33 | 10,5 | 8,0 | 84 | 19 |

LE: N-Acetyl-Lactosamineinheiten (berechnet wie in Beispiel 7)
SA: Sialinsäuregehalt der jeweiligen Isoform.
1 %-Anteil aller Zuckerstrukturen mit zusätzlichen LE-Verlängerungen bezogen auf die Gesamtheit der Zuckerstrukturen (bi + tri + tetra = 100%)

Tabelle 6

| | tetraant. (in %) | repeat[1] (in %) | LE | SA mol/mol | LExSA | spezif. Akt. KU/mg |
|---|---|---|---|---|---|---|
| IF2/A | 99 | 54 | 14,1 | 14 | 197,4 | 396 |
| IF2/B | 97 | 31 | 12,9 | 14 | 180,6 | 330 |
| IF5/A | 64 | 58 | 13,2 | 11 | 145,2 | 206 |
| IF5/B | 55 | 32 | 11,4 | 11 | 125,4 | 112 |
| LE: N-Acetyl-Lactosamineinheiten (berechnet wie im Beispiel 7 beschrieben) SA: Sialinsäuregehalt der jeweiligen Isoform 1 %-Anteil aller Zuckerstrukturen mit zusätzlichen LE-Verlängerungen bezogen auf die Gesamtheit der Zuckerstrukturen (bi +tri +tetra = 100%) | | | | | | |

Tabelle 7

| | tetraant. (in %) | repeat[1] (in %) | LE | SA mol/mol | LexSA | spezif. Akt. KU/mg |
|---|---|---|---|---|---|---|
| IF2 (CHO 2) | 99 | 58 | 14,4 | 14 | 201,6 | 440 |
| IF2 (CHO 3) | 98 | 48 | 13,7 | 14 | 191,8 | 400 |
| IF2 (HeLaS3) | 99 | 24 | 12,9 | 14 | 118,8 | 240 |
| IF5 (CHO 2) | 68 | 48 | 12,9 | 11 | 141,9 | 175 |
| IF5 (CHO 3) | 64 | 39 | 12,0 | 11 | 132,0 | 150 |
| IF5 (HeLaS3) | 70 | 15 | 10,8 | 11 | 119,0 | 60 |
| LE: N-Acetyl-Lactosamineinheiten (berechnet wie in Beispiel 7 beschrieben) SA: Sialinsäuregehalt der jeweiligen Isoform 1 %-Anteil aller Zuckerstrukturen mit zusätzlichen LE-Verlängerungen bezogen auf die Gesamtheit der Zuckerstrukturen (bi + tri + tetra = 100%) | | | | | | |

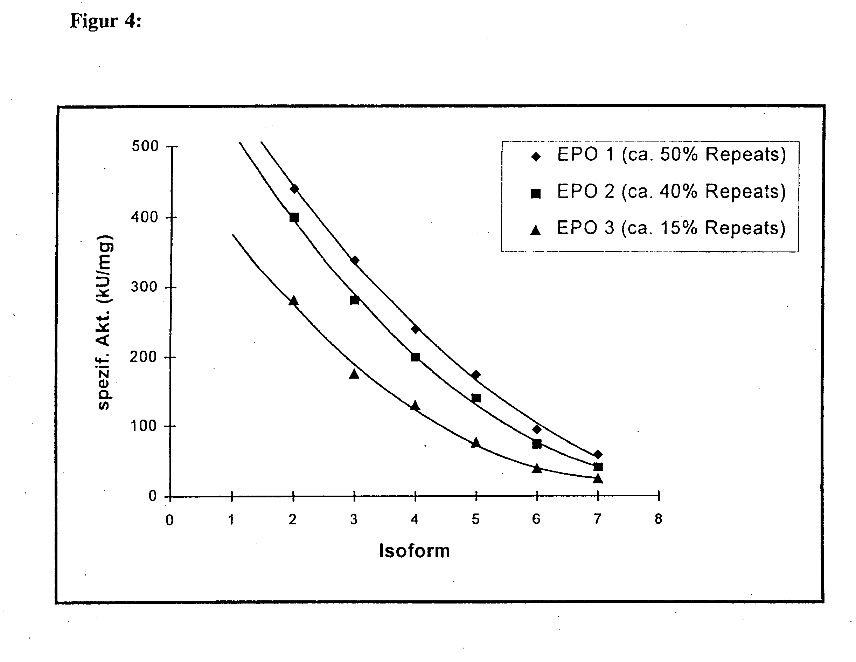

Figur 4 zeigt am Beispiel individueller Isoformen die Abhängigkeit der biologischen Aktivität von EPO vom Anteil an N-gebundenen Kohlenhydratstrukturen mit zusätzlichen N-Acetyl-Lactosamin-Einheiten (Repeats). Die Isoformen wurden aus EPO-Präparationen mit unterschiedlichem Anteil an Repeat-haltigen Kohlenhydratstrukturen isoliert (EPO 1 mit ca. 50%, EPO 2 mit ca. 40% und EPO 3 mit ca. 15% repeathaltiger Strukturen). Die biologische Aktivität entsprechender Isoformen (gleicher Gehalt an Sialinsäuren und etwa gleiche Antennärität) ist um so geringer, je niedriger der Anteil an Repeat-haltigen Kohlenhydratstrukturen in den Isoformen ist. Dieses Merkmal ist von Isoform 2 bis mindestens Isoform 7 zu beobachten.

**Patentansprüche**

1. EPO-Zusammensetzung,
   **dadurch gekennzeichnet,**
   **dass** sie aus glykosylierten EPO-Molekülen besteht, die eine Anzahl von durchschnittlich mindestens 3,7 N-Acetyl-Lactosamin-Einheiten bezogen auf eine N-gebundene Kohlenhydratkette eines EPO-Moleküls bzw. von durchschnittlich mindestens 11,1 N-Acetyl-Lactosamin-Einheiten bezogen auf die Gesamt-N-Glycosilierung eines EPO-Moleküls enthalten, wobei die EPO-Moleküle das Produkt einer Expression endogener DNA in humanen Zellen sind und der Anteil von Kohlenhydratketten mit N-Acetyl-Lactosamin-Repeats bezogen auf die Gesamtzahl von Kohlenhydratketten mindestens 10 % ist, und wobei die EPO-Zusammensetzung eine spezifische Aktivität in vivo von mindestens 175 000 IU/mg Protein aufweist.

2. EPO-Zusammensetzung nach Anspruch 1,
   **dadurch gekennzeichnet,**
   **dass** sie aus glykosilierten EPO-Molekülen besteht, die einen Wert für das Produkt aus der durchschnittlichen Anzahl von N-Acetyl-Lactosamin-Einheiten bezogen auf eine N-gebundene Kohlenhydratkette eines EPO-Moleküls multipliziert mit dem mittleren Sialinsäuregehalt pro Molekül EPO von mindestens 43,3 bzw. mindestens 130 bezogen auf die Gesamt-N-Glycosilierung eines EPO-Moleküls aufweisen.

3. EPO-Zusammensetzung nach Anspruch 2,
**dadurch gekennzeichnet,**
**dass** der Wert des Produkts mindestens 46,7 bezogen auf eine N-gebundene Kohlenhydratkette bzw. mindestens 140 bezogen auf die Gesamt-N-Glycosilierung ist.

4. EPO-Zusammensetzung nach einem der vorhergehenden Ansprüche,
**dadurch gekennzeichnet,**
**dass** sie ein Gemisch aus 2 bis 5 Isoformen umfasst.

5. EPO-Zusammensetzung nach Anspruch 4,
**dadurch gekennzeichnet,**
**dass** sie ein Gemisch aus 3 bis 4 Isoformen umfasst.

6. EPO-Zusammensetzung nach einem der vorhergehenden Ansprüche,
**dadurch gekennzeichnet,**
**dass** sie eine spezifische Aktivität in vivo von mindestens 200 000 IU/mg Protein aufweist.

7. EPO-Zusammensetzung nach einem der vorhergehenden Ansprüche,
**dadurch gekennzeichnet,**
**dass** der mittlere Sialinsäuregehalt pro Molekül mindestens 11 beträgt.

8. EPO-Zusammensetzung nach einem der Ansprüche 1-7,
**dadurch gekennzeichnet,**
**dass** die Kultivierung der Zellen in einem serumfreien Medium erfolgt ist.

9. Pharmazeutisches Präparat,
**dadurch gekennzeichnet,**
**dass** es als Wirkstoff eine EPO-Zusammensetzung nach einem der Ansprüche 1 bis 8 als Wirkstoff gegebenenfalls zusammen mit üblichen pharmazeutischen Verdünnungs-, Hilfs- und Trägermitteln enthält.

**Claims**

1. EPO composition,
characterises in that
it consists of glycosylated EPO molecules which contain a number of at least 3,7 N-acetyl-lactosamine units on average in relation to an N-bound carbohydrate chain of an EPO molecule and of at least 11,1 N-acetyl-lactosamine units on average, respectively, in relation to the total N-glycolysation of an EPO molecule, wherein the EPO molecules are the product of an expression of endogenic DNA in human cells and the percentage of carbohydrate chains with N-acetyl-lactosamine repeats in relation to the total of carbohydrate chains is at least 10%, and wherein the EPO composition has a specific activity in vivo of at least 175 000 IU/mg protein.

2. EPO composition according to claim 1,
**characterised in that**
it consists of glycosylated EPO molecules which have a value for the product of the average number of N-acetyl-lactosamine units in relation to an N-bound carbohydrate chain of an EPO molecule multiplied with the average sialic acid portion per molecule EPO of at least 43.3 and of at least 130 in relation to the total N-glycolysation of an EPO molecule, respectively.

3. EPO composition according to claim 2,
**characterised in that**
the value of the product is at least 46.7 in relation to an N-bound carbohydrate chain and 140 in relation to the total N-glycolysation, respectively.

**4.** EPO composition according to any one of the preceding claims,
**characterised in that**
it comprises a mixture of 2 to 5 isoforms.

**5.** EPO composition according to claim 4,
**characterised in that**
it comprises a mixture of 3 to 4 isoforms.

**6.** EPO composition according to any one of the preceding claims,
characterises in that
it has a specific activity in vivo of at least 200 000 IU/mg protein.

**7.** EPO composition according to any one of the preceding claims,
**characterised in that**
the average sialic acid portion per molecule is at least 11.

**8.** EPO composition according to any one of claims 1-7,
**characterised in that**
the cultivation of the cells has taken place in a serum-free medium.

**9.** Pharmaceutical preparation,
**characterised in that**
it contains an EPO composition according to any one of claims 1 to 8 as active agent optionally in combination with common pharmaceutical diluents, adjuvants and carriers.


**Revendications**

**1.** Composition d'EPO, **caractérisée en ce qu'**elle se compose de molécules d'EPO glycosylées, qui comprennent un nombre d'au moins 3,7, en moyenne, motifs de N-acétyl-lactosamine par rapport à une chaîne d'hydrate de carbone liée à N d'une molécule d'EPO ou un nombre d'au moines 11,1, en moyenne, motifs de N-acétyl-lactosamine par rapport à la N-glycosylation totale d'une molécule d'EPO, les molécules d'EPO étant le produit d'une expression de l'ADN endogène dans des cellules humaines et la proportion de chaîne d'hydrate de carbone avec une répétition de N-acétyl-lactosamine par rapport au nombre total de chaînes d'hydrate de carbone étant de 10 %, et la composition d'EPO présentant une activity spécifique in vive d'au moins 175 000 UI/mg de protéine.

**2.** Composition d'EPO selon la revendication 1, **caractérisée en ce qu'**elle se compose de molécules d'EPO glycosylées, qui présentent une valeur pour le produit issu du nombre moyen de motifs N-acétyl-lactosamine par rapport à une chaîne d'hydrate de carbone liée à N d'une molécule d'EPO, multiplié par la teneur moyenne en acide sialique par molécule d'EPO d'au moins 43,3 ou d'au moins 130 par rapport à la N-glycosylation totale d'une molécule d'EPO.

**3.** Composition d'EPO selon la revendication 2, **caractérisée en ce que** la valeur du product est d'au moins 46,7 par rapport à une chaîne d'hydrate de carbone liée à N ou d'au moins 140 par rapport à la N-glycosylation totale.

**4.** Composition d'EPO selon l'une quelconque des revendication précédentes, **caractérisés en ce qu'**elle comprend un mélange de 2 à 5 isoformes.

**5.** Composition d'EPO selon la revendication 4, **caractérisée en ce qu'**elle comprend un mélange de 3 à 4 isoformes.

**6.** Composition d'EPO selon l'une quelconque des revendications précédentes, **caractérisée en ce qu'**elle présente une activité spécifique in vivo d'au moins 200 000 UI/mg de protéine.

**7.** Composition d'EPO selon l'une quelconque des revendications précédentes, **caractérisée en ce que** la teneur moyenne en acide sialique par molécule est d'au moins 11.

**8.** Composition d'EPO selon l'une quelconque des revendications 1 à 7, **caractérisée en ce que** la culture des cellules s'effectue dans un milieu sans sérum.

9. Préparation pharmaceutique, **caractérisée en ce qu'**elle comprend comme principe actif une composition d'EPO selon l'une quelconque des revendications 1 à 8, le cas échéant conjointement avec des agents de dilution, des auxiliaires et des véhicules pharmaceutiques usuels.

**Figur 1:** Tetraantennäre N-gebundenene Kohlenhydratstruktur mit zusätzlichen N-Acetyl-Lactosamin-Einheiten (Repeats) und Sialinsäuren

Figur 2

relativer Anteil [%]

hochsialyliert    <    Isoformen    >    niedrigsialyliert

—●— Variante E : Glucose
—■— Variante F : Glucose+Galactose+Mannose

Figur 3

Aktivität [kIU/mg]

176

101

Variante E :
Glucose

Variante F :
Glucose
+Galactose
+Mannose

**Figur 4:**

EP 1 037 921 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0148605 B1
- EP 0205564 B1
- EP 0428267 A
- EP 97112640 A
- WO 9635718 A

**In der Beschreibung aufgeführte Nicht-Patentliteratur**

- **MIYAKE et al.** *J. Biol. Chem.,* 1977, vol. 252, 5558-5564
- **TSAO et al.** *Biotech. Bioeng.,* 1992, vol. 40, 1190-1196
- **NIETO et al.** *Anal. Commun.,* 1996, vol. 33, 425-427
- **TRAN et al.** *J. Chromatogr.,* 1991, vol. 542, 459-471
- **BIETOT et al.** *J. Chromatogr.,* 1997, vol. 759, 177-184
- **WATSON et al.** *Anal. Biochem.,* 1993, vol. 210, 389-393
- **IMAI et al.** *Eur. J. Biochem.,* 1990, vol. 194, 457-462
- **TAKEUCHI et al.** *Proc. Natl. Acad. Sci. USA,* 1989, vol. 86, 7819-7822
- **FUKUDA et al.** *Blood,* 1989, vol. 73, 84-89
- **MORIMOTO et al.** *Glycoconjugate J.,* 1996, vol. 13, 1093-1120
- **NADKARNI et al.** *Cancer,* 1969, vol. 23, 64-79
- **RASHEED et al.** *Cancer,* 1973, vol. 33, 1027-1033
- **PUCK et al.** *J. Exp. Meth.,* 1956, vol. 103, 273-284
- **MERKLE.** *Cummings, J. Biol. Chem.,* 1987, vol. 262, 8179-8189
- Linder, Planen und Auswerten von Versuchen. Birkenhäuser Verlag, 1969